# EUROPEAN PATENT SPECIFICATION

(11) **EP 3 448 191 B1**
(45) Date of publication and mention of the grant of the patent: **14.10.2026**
(21) Application number: 17790057.8
(22) Date of filing: 23.03.2017
(51) Int. Cl.: A42B 3/04, A42B 3/08, G08B 5/36, G08B 21/02, A61B 5/11, A61B 5/00, A61N 1/04, A61N 1/36, A61N 1/00, A41D 13/018, A41D 13/05, A63B 71/08, A63B 71/10, A61B 17/135, A63B 69/00, G16H 50/70

(54) **REDUCING BRAIN INJURY BY LIMITING BRAIN MOTION DURING SUDDEN DECELERATION OR ACCELERATION OF THE HEAD**
VERRINGERUNG VON GEHIRNVERLETZUNGEN DURCH BEGRENZUNG DER GEHIRNBEWEGUNG BEIM PLÖTZLICHEN ABBREMSEN ODER BESCHLEUNIGEN DES KOPFES
RÉDUCTION DES LÉSIONS CÉRÉBRALES PAR LIMITATION DU MOUVEMENT CÉRÉBRAL PENDANT UNE DÉCÉLÉRATION OU ACCÉLÉRATION BRUSQUE DE LA TÊTE

(30) Priority: 25.04.2016 US 201662391302 P; 01.11.2016 US 201662496899 P; 23.02.2017 US 201762462906 P
(43) Date of publication of application: 06.03.2019
(73) Proprietor: PreActive Technologies Inc., Kirkland, WA 98033 (US)
(72) Inventor: BRYAN, Vincent E., Quincy, WA 98848 (US); CHING, Randal P., Quincy, WA 98848 (US); BAKER, Daniel Reed, Quincy, WA 98848 (US)
(74) Representative: Mewburn Ellis LLP
(86) International application number: PCT/US2017/023889
(87) International publication number: WO 2017/189132

(56) References cited:
- WO-A1-2017/087556
- US-A- 5 467 766
- US-A- 5 546 609
- US-A1- 2011 082 514
- US-A1- 2014 343 599
- US-B2- 9 226 707

## Description

### CROSS-REFERENCE TO RELATED APPLICATIONS

This application claims priority to U.S. Provisional Application Number 62/391,302 filed on April 25, 2016, U.S. Provisional Application Number 62/496,899 filed on November 1, 2016, and U.S. Provisional Application Number 62/462,906 filed on February 23, 2017.

### TECHNICAL FIELD

The invention relates to medical devices, and more specifically to a system for reducing brain injury in an individual.

### BACKGROUND

Sudden decelerating or accelerating head movements, which may be as a result of direct head impact in sports games (e.g., football), vehicular crashes, accidents (e.g., slips and falls), or on the battlefield, may produce immediate and/or progressive long term devastating brain injury. Such brain injury may be related to excessive brain movement inside of the calvarium which is associated with certain detrimental effects. Histological studies suggest that such injury may take place at the cellular level and adversely affects neuronal axons. As a result, locally damaging chemical and inflammatory responses may be set in motion. These pathophysiological events appear to be related to the sudden acceleration or deceleration of the head as well as the resulting momentum changes to the brain inside of the calvarium at these times.

Conventional efforts have been made to reduce head injury from forceful blows, specifically skull fractures, by means of helmets which have been designed to attenuate the transfer of external energy to the head from a collision. One example includes the design of rigid exterior helmets. Despite this effort brain injuries continue to occur. More recent helmet designs using flexible exterior, shock absorbing, multilayer helmets with novel flexible resilient interior baffles may offer better protection to the skull. However, these conventional approaches do not offer a means to limit brain motion inside of the skull, and as such, brain injury as a result of forceful blows to the head remains a concern.

Patent application US 2014/343599 A1 discloses a system for reducing the damaging effects of radiant energy, blast, or concussive events includes applying pressure to at least one jugular vein to reduce the egress of blood from the cranial cavity during or before the incidence of the imparting event.

### SUMMARY

The invention defines a system for reducing a severity of a brain injury incurred by an individual due to a change in head acceleration of the individual, as described in claim 1.

Embodiments of the invention include a brain injury reduction system that provides a protective measure to an individual that is currently experiencing or will undergo acceleration or deceleration of the head (e.g., resulting from impact or a blast pressure wave from an explosive device). A protective measure provided by the brain injury reduction system reduces an individual's brain movement relative to the calvarium during times of sudden decelerating or accelerating head movements, thereby reducing the severity of an injury that would be incurred by an individual's brain. Specifically, the brain injury reduction system reduces brain injury by decreasing venous drainage (e.g., maintaining or increasing a central venous pressure) from the brain at or immediately prior to the sudden head acceleration or deceleration. The reduced venous drainage corresponding to a maintained central venous pressure reduces the movement of the brain within the calvarium when a sudden deceleration or acceleration of the head occurs. This reduction in brain motion within the calvarium, in turn, results in less distortion or deformation of the brain tissues (straining or stretching of the tissues).

Generally, venous drainage from the intracranial compartment occurs via the jugular veins, and more specifically the internal jugular veins (IJV), when an individual is supine and the paravertebral venous plexus (PVP), also referred to as the vertebral venous plexus (VP) when upright. When an impact to the head (or blast pressure wave) occurs, increased venous drainage from the intracranial compartment may occur, thereby increasing the intracranial space within the head that allows the brain to displace within the calvarium. As such, a reduction in venous drainage from the intracranial compartment could reduce the intracranial space made available in the head and thereby reduce brain movement that is a consequence of the sudden acceleration or deceleration movement of the individual's head. Reduction of venous drainage through the PVP can be achieved by glottis closure (e.g., through a gag reflex) or through a valsalva maneuver (or through other mechanisms that reduce this venous drainage). Reduction of venous drainage through one or both of the IJV and external jugular veins (EJV) can be achieved through external compression of the IJV and EJV, respectively.

The brain injury reduction system decreases drainage of venous blood out of the intracranial compartment through one or both of the PVP and the IJVs at or immediately before the occurrence of a sudden acceleration or deceleration of an individual's head. Specifically, the brain injury reduction system includes components that may be worn by the individual such as a wearable head-impact and/or position-tracking sensing device (e.g., miniature sensors mounted into a helmet, mouthguard, or headband) as well as one or more wearable actuation devices that are each configured to reduce venous drainage through either the IJVs or PVP. A wearable actuation device can be configured to reside around the neck of the individual and expand to externally compress the IJVs when required. A wearable actuation device can be configured to stimulate a gag reflex or a valsalva-like maneuver. Each of the devices are communicatively coupled with one another such that each of the one or more wearable actuation devices can instantaneously provide the protective measure in response to a detected event, such as an impending collision, by the wearable sensing device. The brain injury reduction system can subsequently terminate the protective measure after the occurrence of the collision or after a pre-determined endpoint occurs.

### BRIEF DESCRIPTION OF THE DRAWINGS

The disclosed embodiments have advantages and features which will be more readily apparent from the detailed description, the appended claims, and the accompanying figures (or drawings). A brief introduction of the figures is below.
Figure (FIG.) 1A illustrates an overall system environment for providing a protective measure for reducing brain injury in an individual, in accordance with an embodiment.
FIGs. 1B and 1C each illustrates components of the brain injury reduction system worn by an individual, in accordance with an embodiment.
FIG. 2 illustrates a right side view of a wearable device in communication with an actuation device, in accordance with an embodiment.
FIGs. 3A-C illustrates an example actuation device for compressing the jugular veins of an individual, in accordance with an embodiment.
FIGs. 4A-4C each illustrates an example actuation device for inducing a gag reflex in an individual, in accordance with an embodiment.
FIGs. 5A-5D each illustrates an example actuation device for inducing a valsalva-like maneuver, in accordance with an embodiment.
FIG. 6 illustrates an example block diagram depicting the system architecture of a computing system, in accordance with an embodiment.
FIG. 7 illustrates an example interaction diagram for providing a protective measure for reducing brain injury in an individual, in accordance with an embodiment.

The Figures (FIGs.) and the following description relate to preferred embodiments by way of illustration only. It should be noted that from the following discussion, alternative embodiments of the structures and methods disclosed herein will be readily recognized as viable alternatives that may be employed without departing from the scope of the invention that is defined by the appended claims.

### DETAILED DESCRIPTION

Reference will now be made in detail to several embodiments, examples of which are illustrated in the accompanying figures. It is noted that wherever practicable similar or like reference numbers may be used in the figures and may indicate similar or like functionality. For example, a letter after a reference numeral, such as "320a," indicates that the text refers specifically to the element having that particular reference numeral. A reference numeral in the text without a following letter, such as "320," refers to any or all of the elements in the figures bearing that reference numeral (e.g. "actuating component 320" in the text refers to reference numerals "actuating component 320a" and/or "actuating component 320b" in the figures).

### OVERALL SYSTEM ENVIRONMENT

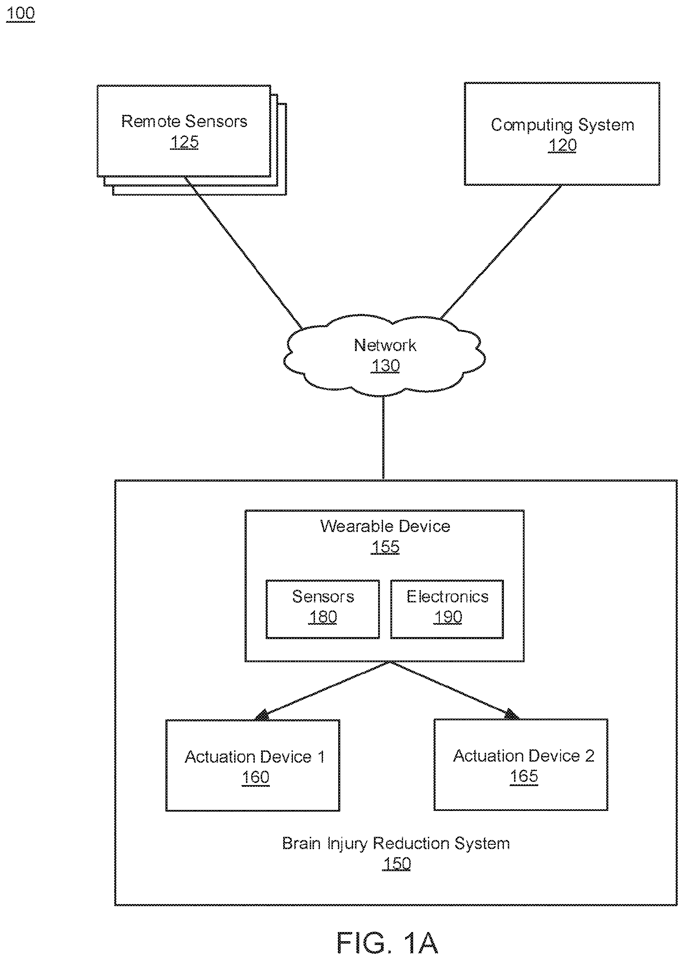

FIG. 1A illustrates an overall system environment 100 for providing a protective measure for reducing brain injury in an individual, in accordance with an embodiment. Generally, the overall system environment 10 includes a brain injury reduction system 150, a computing system 120, and remote sensors 125 that are interconnected through a network 130. As shown in FIG. 1A, the brain injury reduction system 150 may further include a wearable device 155, an actuation device 1 (160), and an actuation device 2 (165).

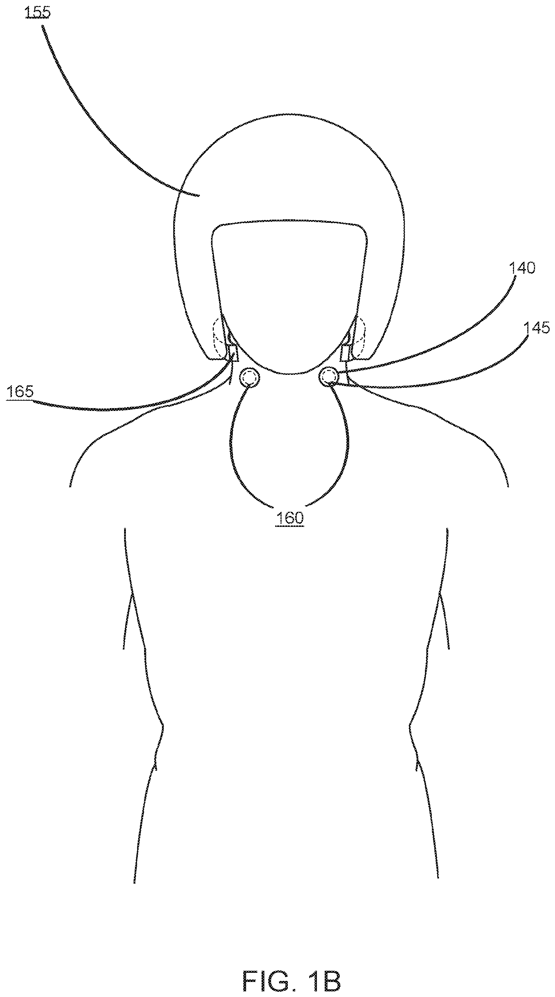

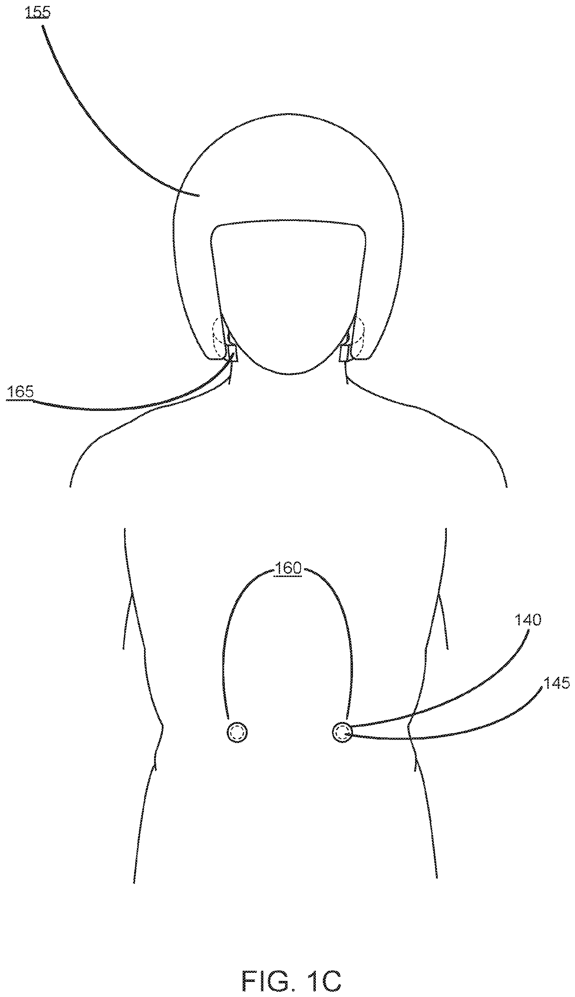

Although the illustrated environment 100 may include the elements shown in FIG. 1, in other embodiments the environment 100 may include different elements (e.g., additional or fewer elements). In one embodiment, the system environment 100 may include multiple brain injury reduction systems 150 in communication with one another, the computing system 120, and remote sensors 125 through the network 130. In some embodiments, the system environment 100 does not include a computing system 120 and/or the remote sensors 125.

### Example Brain Injury Reduction System

As shown in FIG. 1A, a brain injury reduction system 150 includes a wearable device 155 as well as one or more actuation devices 160, 165. In various embodiments each of the wearable device 155 and actuation devices 160,165 are wearable and configured to be worn by a particular individual. As such, a brain injury reduction system 150 is responsible for reducing brain injury in a particular individual. In various embodiments, each of the wearable device 155, actuation device 1 (160), and actuation device 2 (160) includes wearable housings that contain components of each of the wearable device 155, actuation device 1 (160), and actuation device 2 (160).

Notably, the subsequent disclosure that describes each of the wearable device 155, actuation device 1 (160) and actuation device 2 (165) will refer to each device in one of a rest state or an actuated state. The rest state refers to the default state of each device when a protective measure is not provided. Each device can transition to an actuated state when a protective measure is needed. Specifically, when each device is in an actuated state, the brain injury reduction system 150 maintains the central venous pressure in the brain in the individual, thereby reducing brain injury due to decelerating or accelerating head motion (e.g., resulting from head impact or a pressure wave from an explosive device).

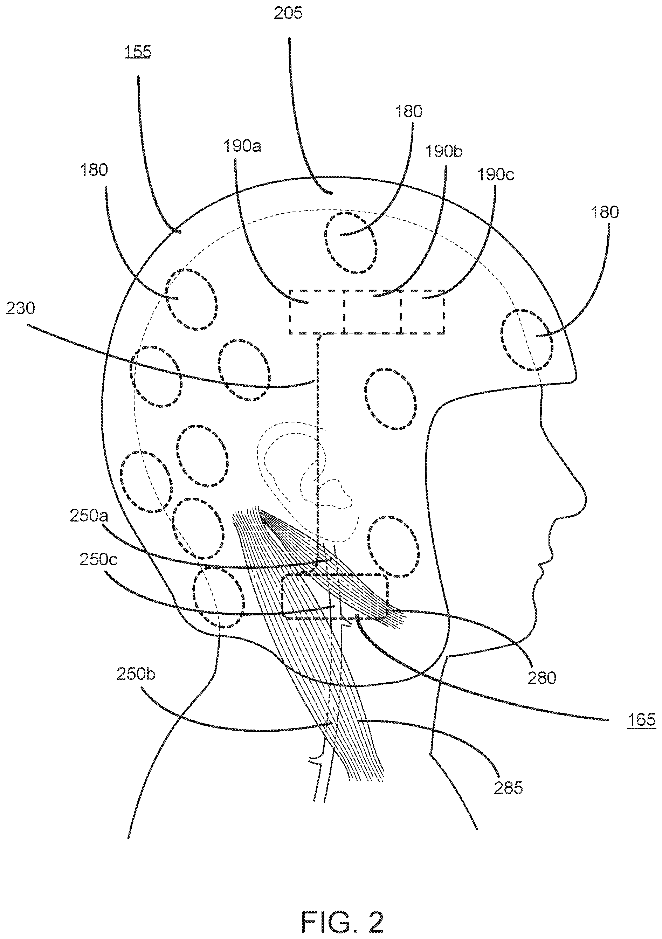

In one embodiment, the wearable device 155 is headwear, such as a helmet, worn by an individual. In other embodiments, the wearable device 155 can be worn on another anatomical location (e.g., chest, neck, hip, extremities such as arms or legs, and the like) of the individual. The wearable device 155 can be a wearable sensing device that includes sensors 180 and electronics 190. The sensors 180 of the wearable device 155 can be configured to detect an occurring or impending collision. The electronics 190 of the wearable device 155 may include one or more of a power source, processor, or communication hardware. Therefore, the electronics 190 of the wearable device 155 enable the wearable device 155 to communicate with the computing system 120 and/or remote sensors 125 through the network 130. Additionally, the electronics 190 of the wearable device 155 can enable the wearable device 155 to communicate with the actuation devices 160 and 165 (e.g., through Bluetooth, near field communication (NFC), WiFi, 2G, 3G, 4G, or long-term evolution (LTE)). Further description regarding the sensors 180 and electronics 190 of the wearable device 155 are described below in reference to FIG. 2.

Actuation device 1 (160) and an actuation device 2 (165) are each configured to stimulate and cause a type of an anatomical or physiological response in an individual that assists in the reduction of brain injury. In some embodiments, the brain injury reduction system 150 includes a single actuation device 160. In other embodiments, the brain injury reduction system 150 includes multiple actuation device 1 (160) that each causes or reinforces a first type of anatomical or physiological response in the individual and/or multiple actuation device 2 (165) that each causes a second type of anatomical or physiological response in the individual.

Reference is now made to FIG. 1B and FIG. 1C, which each illustrates components of the brain injury reduction system 150 worn by an individual.
Specifically, FIG. 1B depicts a wearable device 155 as a helmet, actuation device 1 (160), and actuation device 2 (165) that is configured to circumferentially surround a portion of the neck of the individual.
In some embodiments, a wearable device 155 can be a wearable garment such as a shirt (e.g., football jersey), pants, shoes, protective wear (e.g., brace, pads), and the like.

Referring first to actuation device 2 (165) shown in FIG. 1B, the actuation device 2 (165) may be situated around the neck of the individual so as to be in close proximity to the skin of the individual under which the IJVs reside. In some embodiments, the actuation device 2 (165) is physically coupled to the wearable device 155 through one of straps, buckles, adhesives, buttons, and the like. In various embodiments the actuation device 2 (165) is removably coupled to wearable device 155. In other embodiments, the actuation device 2 (165) is a standalone device and separate from the wearable device 155.

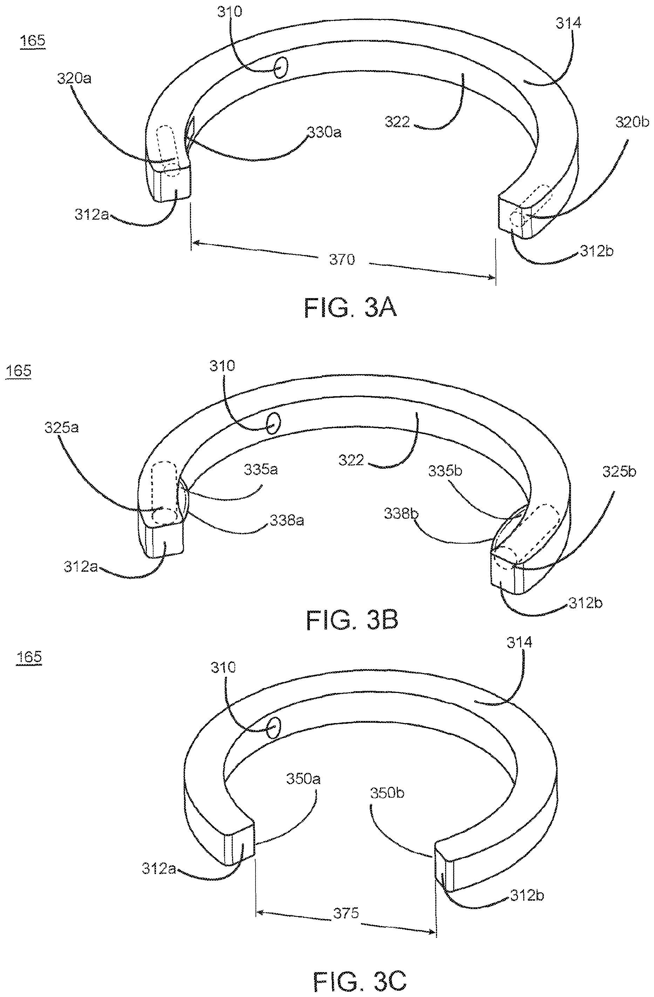

When in an actuated state, actuation device 2 (165), may change its configuration to physically compress the IJVs, thereby reducing venous drainage through the IJV. For example, actuation device 2 (165) may include protrusions that extend from the housing of the actuation device 2 (165) to externally compress the IJVs. An example actuation device 2 (165) is described in further detail below in regards to FIG. 3.

Referring now to actuation device 1 (160), it is configured to cause one of a gag reflex or a valsalva-like maneuver in the individual when in an actuated state. Therefore, actuating the actuation device 1 (160) causes a reduction in venous drainage through the PVP. As depicted in FIG. 1B, actuation device 1 (160) can include a pair of stimulation structures such as electrode leads 145 in contact with the skin of the individual. These may be neuromuscular stimulation structures that are configured to stimulate a nerve and/or a muscle. Additionally, the electrode leads 145 may be located on or within an electrode housing 140. As an example, an electrode housing 140 may be an electrode pad that houses the electrode leads 145 such that the electrode housing 140 is in contact with the individual's skin.

In some embodiments, the electrode leads 145 or the electrode housing 140 is attached to the skin through any one of adhesives or gels. Such adhesives or gels can hold the electrode leads 145 or the electrode housing 140 in contact with the skin of the individual and, in some embodiments, can further improve the conductivity of the skin. In various embodiments, instead of a pair of electrode leads 145, a single electrode lead 145 in contact with the skin of the individual is sufficient to provide a stimulation. The electrode leads 145 may be connected (e.g., wired) to a power source such as a minimal battery such that the actuation device 1 (160) remains non-intrusive.

As depicted in FIG. 1B, the electrode leads 145 and the electrode housing 140 of the actuation device 1 (160) are spherical in shape. However, in other embodiments, the electrode leads 145 and electrode housing 140 may be square, rectangular, triangular, oval, hexangular, or another polygon in shape. In various embodiments, the electrode leads 145 are between 1 millimeter and 10 millimeters in diameter. In other embodiments, the electrode leads 145 may be larger between 10 millimeters and 10 centimeters in diameter.

Each electrode lead 145 may be configured to provide a transcutaneous stimulation to a corresponding nerve located beneath the skin. Therefore, actuation of the electrode leads 145 (e.g., providing an electric input) can stimulate a nerve and result in a desired anatomic response. Specifically, actuation device 1 (160) depicted in FIG. 1B may contact the skin of the individual located external or near to the laryngeal nerves and therefore causes a glottic closure as a result of stimulating the laryngeal nerves.

Referring now to FIG. 1C, according to the invention, the actuation device 1 (160) may be placed in contact with the skin of the individual at a different anatomical location. Specifically, as shown in FIG. 1C, actuation device 1 (160) may include stimulation structures such as electrode leads 145 that are in contact with the skin of the individual located above or near the thoraco-abdominal nerves. Also depicted in FIG. 1C are electrode housings 145 that are configured to house the electrode leads 140. Actuation of the actuation device 1 (160) causes a valsalva-like maneuver as a result of stimulating the thoraco-abdominal nerves or the rectus abdominal muscles directly.

Although FIG. 1B and FIG. 1C each depict two different positions of actuation device 1 (160), in various embodiments, the actuation device 1 (160) may include a first set of electrode leads 145 in contact with the skin of the individual that is configured to stimulate the laryngeal nerves and a second set of electrode leads 145 in contact with the skin of the individual that is configured to stimulate the thoraco-abdominal nerves. As such, actuation of actuation device 1 (160) can cause both a gag reflex and a valsalva-like maneuver.

The actuation device 1 (160) and the actuation device 2 (165) may each contain electronics that enable each actuation device 160 and 165 to communicate with the wearable helmet 155. More specifically, the actuation device 1 (160) and the actuation device 2 (165) can receive input from the wearable helmet 155 through the electronics that cause each actuation device 160 and 165 to actuate. Altogether, actuation of one or both of actuation device 1 (160) and actuation device 2 (165) maintains or increases the venous pressure within the individual's brain due to a reduction in venous drainage. Additional embodiments of actuation devices 160 and 165 are described in further detail below in regards to FIG. 2-5.

### Example Remote Sensors

Referring back to FIG. 1, the system environment may include remote sensors 125 that can be a data gathering sensor. For example, a remote sensor 125 may be a sensor capable of capturing signals including, but not limited to, electromagnetic radiation and acoustic signals. For example, a remote sensor 125 may be a camera with a charge coupled device that captures an image by capturing individual pixels of light intensities. As another example, a remote sensor 125 may be in an aerial vehicle (e.g., a drone) that can capture signals.

In one embodiment, the remote sensors 125 may gather data in relation to the wearable device 155 of the brain injury reduction system 150. More specifically, the remote sensors 125 gather the position and orientation of the wearable device 155 prior, during, and subsequent to a collision which may cause head impact. Additionally, the remote sensors 125 gathers data corresponding to the collision which can include the speed of collision and estimated overall force imparted due to collision.

In various embodiments, the remote sensors 125 can gather data corresponding to multiple brain injury reduction systems 150. As a specific example, if the system environment 100 is employed for a football game, a first brain injury reduction system 150 can be associated with a first player and a second brain injury reduction system 150 can be associated with a second player. Therefore, the remote sensors 125 can track data corresponding to a collision between the first player and the second player. As an additional example, the remote sensors 125 can track the positions and/or speeds of the first player and the second player and detect an impending collision given their tracked positions and/or speeds.

In various embodiments, the remote sensors 125 can be in communication with the computing system 120. Therefore, the remote sensors 125 provide the gathered data corresponding to a wearable device 155 or the gathered data corresponding to a collision to the computing system 120 for analysis.

### Example Computing System

A computing system 120 is in communication with the remote sensors 125 and one or more brain injury reduction systems 150 through the network 130. A computing system 120 may be a server computer, a client computer, a personal computer (PC), a tablet PC, a set-top box (STB), a smartphone, an internet of things (IoT) appliance, a network router, switch or bridge, or any machine capable of executing instructions (e.g., program code or software) stored in a memory that specify actions to be taken by that computing system 120.

In various embodiments, the computing system 120 receives data captured by one or both of the remote sensors 125 and data captured by the brain injury reduction system 150. The computing system 120 analyzes the data to improve upon the response to future collisions. As an example, the computing system 120 can more accurately determine whether a protective measure is needed. In some scenarios, a protective measure is needed when an individual is currently experiencing or will experience a collision that is unexpected. Alternatively, a protective measure is unneeded when an individual has anticipated an occurring collision or is anticipating for an impending collision.

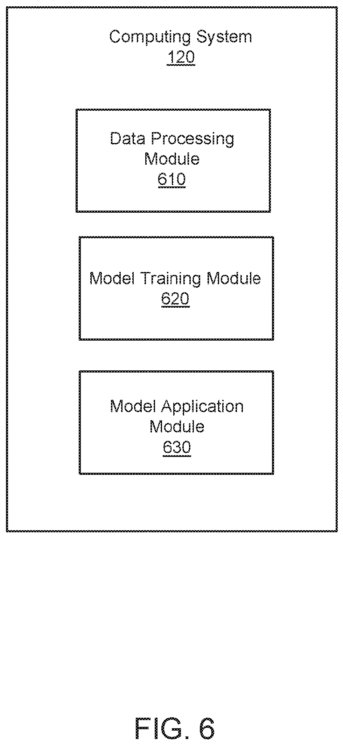

In one embodiment, when a subsequent occurring or impending collision is detected, the computing system 120 receives data corresponding to that occurring or impending collision and determines whether a protective measure is needed. In other embodiments, the computing system 120 provides analysis information to the wearable device 155 such that when a subsequent occurring or impending collision is detected, the wearable device 155 can perform the analysis to determine whether a protective measure is needed. Further discussion regarding the computing system 120 is described below in reference to FIG. 6.

### Example Network

The network 130, which can be wired, wireless, or a combination thereof, enables communications between the brain injury reduction system 150, the computing system 120, and the remote sensors 125. The network 130 may include the Internet, a local area network (LAN), virtual LAN (VLAN) (e.g., with VPN), wide area network (WAN), or other network. In one embodiment, the network 130 uses standard communications technologies and/or protocols, such as Hypertext Transfer Protocol (HTTP), Transmission Control Protocol/Internet Protocol (TCP/IP), Uniform Resource Locators (URLs), and the Doman Name System (DNS). In another embodiment, the brain injury reduction system 150, the computing system 120, and the remote sensors 125 can use custom and/or dedicated data communications technologies instead of, or in addition to, the ones described above.

### EXAMPLE WEARABLE DEVICE

FIG. 2 illustrates a right side view of a wearable device 155 in communication with an actuation device 2 (165), in accordance with an embodiment. The wearable device 155 can include sensors 180 and electronics 190. In some embodiments, the wearable device 155 includes wired connections 230 that enable the electronics 190 of the wearable device 155 to communicate with the actuation device 2 (165).

Referring to the sensors 180 of the wearable device 155, FIG. 2 depicts multiple sensors 180 distributed throughout the wearable device 155. In some embodiments, the sensors 180 are housed on or within a wearable housing 205 of the wearable device 155. For example, the wearable housing 205 may be the rigid helmet itself or a component within the rigid helmet. In various embodiments, the sensors 180 are evenly distributed or within the wearable housing 205 of the wearable device 155. In other embodiments, the concentration of sensors 180 at a location on or within the housing 205 of the wearable device 155 may be different where the individual may be more susceptible to brain injury. For example, as depicted in FIG. 2, the posterior of the wearable device 155 may include a higher concentration of sensors 180 in order to more accurately detect an occurring or impending collision that may be approaching from the posterior of a vulnerable individual.

The sensors 180 may be circular in shape. However, in various embodiments, the sensors 180 may be square, rectangular, triangular, oval, hexangular, or another polygon in shape. In some embodiments, the size of each sensor 180 can range from 1 millimeter to 5 centimeters or more in both size and thickness. In other embodiments, the size of the sensor 180 is larger and maintains its function of capturing a signal.

In one embodiment, the sensors 180 may be located on the external surface of the housing of the wearable device 155. In this scenario, the sensors 180 may be pressure sensors configured to detect changes in acoustics (e.g., pressure waves) in the vicinity of the sensors 180. As another example, the sensors 180 may be electromagnetic sensors configured to detect changes in electromagnetic radiation (e.g., energy across the electromagnetic spectrum such as light or magnetic waves) in the vicinity of the sensors 180. Generally, the changes in acoustics and/or changes in electromagnetic radiation may arise from an approaching object. Therefore, the sensors 180 on the external surface of the wearable device 155 can detect an impending collision due to the approaching object. In various embodiments, the sensors 180 detect an impending collision between 0 to 1 seconds beforehand. In some embodiments, the sensors 180 detect an impending collision between 100 to 500 milliseconds beforehand. In various embodiments, the sensors 180 detect an impending collision between 200 and 300 milliseconds beforehand.

In some embodiments, the sensors 180 may be located on an internal surface (e.g., on an inner most surface or within two layers) of the housing 205 of the wearable device 155. In this scenario, the sensors 180 may be one or both of accelerometers or gyroscopes that are configured to gather data regarding the orientation and/or position of the wearable device 155. An accelerometer can gather the linear kinematics (e.g., acceleration and velocity) along one or more axes of the wearable device 155 whereas the gyroscope can gather the rotational kinematics (e.g., acceleration and velocity) along one or more rotational axes of the wearable device 155.

In various embodiments, a wearable device 155 can include sensors 180 located on both the external surface and the internal surface of the wearable device 155. For example, the wearable device 155 can include sensors 180 (e.g., pressure or electromagnetic sensors) configured to detect an impending collision from an approaching object as well as sensors 180 (e.g., accelerometer and gyroscope) configured to detect the orientation and/or position of the wearable device 155.

Referring now to the electronics 190 of the wearable device 155, the electronics 190 may include one or more of communication electronics 190a (e.g., that enable communication via Bluetooth, NFC, WiFi, 2G, 3G, 4G, or LTE), processor 190b, and power source 190c (e.g., battery). In various embodiments, the communication electronics 190a, the processor 190b, and power source 190c can be distributed at different locations around the wearable device 155. Each of the communication electronics 190a, processor 190b, and power source 190c can be housed within the housing 205 of the wearable device 155. Generally, the power source 190c provides power to the sensors 180, communication electronics 190a, and processor 190b of the wearable device 155.

Specifically, the communication electronics 190a enable the wearable device 155 to communicate with the remote sensors 125 and the computing system 120 through the network 130. Additionally, the communication electronics 190a enable the wearable device 155 to communicate with the actuation device 1 (160) and the actuation device 2 (165).

In one embodiment, the communication electronics 190a of the wearable device 155 receives a trained machine-learning model to be applied by the processor 190b to determine whether a protective measure is required. In another embodiment, the communication electronics 190a of the wearable device 155 receives instructions from the computing system 120 indicating that a protective measure is required. Thus, the communication electronics 190a of the wearable device 155 can provide an input to one or both of the actuation device 1 (160) and the actuation device 2 (165) to transition from a rest state to an actuated state in order to reduce venous drainage from the individual's head. Additionally, the communication electronics 190a of the wearable device 155 can provide an input to one or both of the actuation device 1 (160) and the actuation device 2 (165) to cease actuation and transition back into a rest state.

In some embodiments, the wearable helmet 155 includes wired connections 230 such that the communication electronics 190a can directly communicate with actuation device 2 (165) through the wired connection 230. A wired connection 230 may be preferable to reduce the latency as compared to wireless communication methods.

Referring now to the processor 190b of the wearable helmet 155, in various embodiments, the processor 190b can be configured to execute instructions (e.g., code) that determine whether a protective measure is needed. Therefore, in this scenario, the processor 190b of the wearable helmet 155 receives a trained machine-learning model from the computing system 120 to be used to determine whether a protective measure is needed. For example, the processor 190b of the wearable helmet 155 can receive sensor-gathered data from the sensors 180 of the wearable helmet 155 and apply the sensor-gathered data as input to the trained machine-learning model. More specifically, the trained machine-learning model analyzes the sensor data (e.g., orientation and/or position of the wearable helmet 155 and/or impending collision information) and outputs whether the protective measure is to be performed. As an example, the machine-learning model may be trained to classify impending collisions that a vulnerable individual is not expecting (e.g., from the posterior of the individual) as requiring a protective measure. Therefore, if a protective measure is deemed necessary by the machine learning model, the processor 190b instructs the communication electronics 190a to transmit inputs to actuate one or both of actuation device 1 (160) and actuation device 2 (165).

In various embodiments, the processor 190b further detects an endpoint and subsequently instructs the communication electronics 190a to provide an input to one or both of actuation device 1 (160) and actuation device 2 (165) to transition from an actuated state back to the rest state. Therefore, the individual that was provided the protective measure need not experience prolonged physical compression on the IJV and/or a prolonged induced gag reflex and/or valsalva-like maneuver.

In various embodiments, the detected endpoint may be a collision. For example, a preventive measure can initially be provided in response to an impending collision. Once the impending collision occurs, the processor 190b detects the occurred collision as an endpoint and provides input such that the actuation device 1 (160) and/or actuation device 2 (165) transitions back to the rest state. In some embodiments, the endpoint is the detection of multiple collisions. For example, a first collision may correspond to a collision with a moving object (e.g., a collision with another football player) whereas a second collision may correspond to a collision with an inanimate object (e.g., the ground). Therefore, the protective measure may be held and provided through the multiple collisions. Once the processor 190b receives notification that multiple collisions were detected, the processor 190b can provide input to transition the actuation device 1

(160) and/or actuation device 2 (165) back to the rest state. In some embodiments, the detected endpoint is a threshold duration of time. For example, the duration may be several seconds (e.g., 3 seconds, 5 seconds, or 10 seconds). After the threshold duration of time has passed, the processor 190b causes the actuation device 1 (160) and/or actuation device 2 (165) to transition back to the rest state.

### EXAMPLE ACTUATION DEVICE FOR CLOSING INTERNAL JUGULAR VEINS

Referring to FIG. 2, the actuation device 2 (165) may be located on the interior of the housing 205 wearable device 155. Specifically, the actuation device 2 (165) is located between the wearable device 155 and the skin of the individual that is external to the jugular veins. In some embodiments, the actuation device 2 (165) is configured to compress one or both of the left/right internal jugular veins (IJV) and the external jugular veins (EJV). Although FIG. 2 depicts a right side view of the actuation device 2 (165) that is configured to externally compress the one or both of the right IJV and right EJV, one skilled in the art can readily understand that the actuation device 2 (165) can be configured to provide bilateral compression to both the right and left IJV/EJV. Therefore, the subsequent description refers to both the right and left IJV/EJV.

FIG. 2 further depicts the anatomical structure of the right IJV of the human individual. Specifically, the digastric muscle 280 is positioned superficially relative to a first portion 250a of the IJV. In other words, the digastric muscle 280 covers and prevents external access through the skin to the first portion 250a of the IJV. Additionally, the sternocleidomastoid muscle 285 is positioned superficially relative to a second portion 250b of the IJV. In other words, the sternocleidomastoid muscle 285 covers and prevents external access through the skin to the second portion 250b of the IJV. As shown in FIG. 2, the first portion 250a of the IJV is proximal to the individual's brain relative to the second portion 250b of the IJV. A third portion 250c of the IJV is externally accessible by compression through the skin. Therefore, the actuation device 2 (165) can be in contact with the skin of the individual located external to the third portion 250c of the IJV such that the actuation device 2 (165), when actuated, physically compresses and reduces venous drainage through the IJV.

Reference is now made to FIGs. 3A-3C, which each illustrates an example actuation device 2 (165) for compressing the IJVs of an individual, in accordance with an embodiment. More specifically, FIG. 3A depicts the actuation device 2 (165) in a rest state. The actuation device 2 (165) may be composed of a body, otherwise referred to as the housing 314. The housing 314 may include a first end 312a and a second end 312b. The actuation device 2 (165) includes communication electronics 310 that enable the actuation device 2 (165) to communicate with the wearable device 155 through one of Bluetooth, near field communication (NFC), WiFi, 2G, 3G, 4G, LTE, or other wireless or wired communication methods. In some embodiments, the actuation device 2 (165) further includes stimulation structures. In FIG. 3A, these stimulation structures include an extendable structure 330a located on an inner surface 322 of the housing 314 of the actuation device 2 (165) near the first end 312a. Of note, the actuation device 2 (165) may also include an extendable structure located on an inner surface 322 of the housing 314 of the actuation device 2 (165) near the second end 312b which is not shown in FIG. 3A. Additionally, the actuation device 2 (165) can include one or more actuating components 320.

Referring first to the communication electronics 310 of the actuation device 2 (165), they enable the actuation device 2 (165) to communicate with the wearable device 155. Specifically, the communication electronics 310 of the actuation device 2 (165) receives input from the wearable device 155 after a protective measure is deemed necessary. Therefore, the actuation device 2 (165) can transition from a rest state, as depicted in FIG. 3A, to an actuated state as is depicted in FIG. 3B and 3C. Alternatively, the communication electronics 310 can receive input from the wearable device 155 after an endpoint is detected. Therefore, the actuation device 2 (165) can transition from the actuated state back to the rest state. Although FIG. 3A depicts the communication electronics 310 as being located on the inner surface 322 of the housing 314 of the actuation device 2 (165), in various embodiments, the communication electronics 310 can be located on another surface of the housing 314 or internally within the housing 314 of the actuation device 2 (165).

In various embodiments, the housing 314 and the extendable structures 330 of the actuation device 2 (165) are each constructed from a solid polymer including, but not limited to, polystyrene, polypropylene, polyurethane, nylon, leather, rubber, and the like. Therefore, any component of the actuation device 2 (165), and more specifically the extendable protrusions 330, that contact the individual would retain its structure, thereby enabling the actuation device 2 (165) to physically compress the IJVs.

The actuation device 2 (165) may have dimensional characteristics that are tailored for the individual that wears the actuation device 2 (165). Specifically, the first end 312a and the second end 312b of the actuation device 2 (165) may be a pre-determined distance 370 apart. The pre-determined distance 370 can be selected according to the anatomical characteristics of the individual (e.g., neck size of the individual). Additionally, the housing 314 of the actuation device 2 (165) may have a curvature that is also selected according to the anatomical characteristics of the individual. For example, the curvature of the housing 314 can be designed to allow for about a 1 centimeter gap between actuation device 2 (165) and the neck of the individual when the actuation device 2 (165) is in a rest position. In other embodiments, the curvature of the housing 314 is designed to allow for a gap anywhere from 0.1 mm to 100 mm between actuation device 2 (165) and the neck of the individual when the actuation device 2 (165) is in a rest position. As such, the actuation device 2 (165) need not contact the skin of the individual in the rest position until a protective measure is determined to be required, which can trigger the actuation device 2 (165) to transition into the actuated state. Generally, the pre-determined distance 370 and the curvature of the housing 314 are selected such that the internal surface 322 of the actuation device 2 (165) remains a distance away from the skin of the individual when the actuation device 2 (165) is in the rest state. In some embodiments the internal surface 322 of the actuation device (165) may rest on the skin of the individual.

As described above, the housing 314 of the actuation device 2 (165) may include a first extendable protrusion 330a and a second extendable protrusion 330b on an internal surface 322 of the actuation device 2 (165). Additionally, the housing 314 of the actuation device 2 (165) can further house multiple actuating components 320. In some embodiments, each actuating component 320 is located within the actuation device 2 (165) as is indicated by the dotted outline structure in FIG. 3A. In various embodiments, the housing 314 may only include a single actuating component 320 that is configured to trigger both extendable protrusions 330a and 330b.

In the rest state, each extendable protrusion 330 can sit flush with the internal surface of the actuation device 2 (165). Therefore, in the rest state, each extendable protrusion 330 is unlikely to unintentionally contact the individual's skin and the underlying IJV. Each extendable protrusion 330 can transition from a rest state (e.g., flush to the inner surface 322) to an actuated state (e.g., extended outward from the inner surface 322) due to actuating components 320 of the actuation device 2 (165).

Each actuating component 320 of the actuation device 2 (165) can be triggered when the communication electronics 310 of the actuation device 2 (165) receives the input indicating a need for a protective measure from the wearable device 155. As shown in FIG. 3A, the actuation device 2 (165) includes two actuating components 320. A first actuating component 320a can be located within the housing 314 near or at the first end 312a whereas the second actuating component 320b can be located within the housing 314 near or at the second end 312b.

An actuating component 320 can provide one of a mechanical, chemical, or electrical inputs to cause an extendable protrusion 330 to change its configuration. This may occur in response to the communication electronics 310 receiving an input from the wearable device 155. For example, the actuating component 320 may be a physical structure that displaces inward. As such, the actuating component 320 mechanically displaces the extendable protrusion 330 to protrude inward from the inner surface 322. As another example, the actuating component 320 may store expandable fluids in separate compartments or cartridges such as gasses, liquids, gels, and the like. Thus, the expandable fluids can be mixed when desired that causes an increase in volume. The increase in volume can cause the extendable protrusions 330 to change their configuration and protrude inward from the inner surface 322. As another example, the actuating component 320 may include electrical components that provide an electrical input that cause the extendable protrusions 330 to change their configuration.

FIG. 3B depicts one embodiment of the actuation device 2 (165) in an actuated state. Specifically, each actuating component 325a and 325b may now be in an actuated state, as indicated by their larger size in FIG. 3B as compared to their counterparts 320a and 320b shown in FIG. 3A. Each actuated actuating component 325 causes a corresponding change to achieve an extended protrusion 335a and 335b. For example, each extended protrusion 335 is extended inward from the inner surface 322 when in an actuated state. Therefore, the first extended protrusion 335a can physically compress the right EJV and/or IJV whereas the second extended protrusion 335b can physically compress the left EJV and/or IJV. In some embodiments, the actuation device 2 (165) can have two extended protrusions 335 on either side (total of four extended protrusions 335), each extended protrusion configured to physically compress one of the right EJV, right IJV, left EJV, and left IJV.

As shown in FIG. 3B, each extended protrusion 335 may possess a convex rounded edge 338 that extends inward from the inner surface 322. As such, the rounded edge 338 of the extended protrusion 335 physically compresses the EJV and/or IJV of the individual. In other embodiments, the extended protrusion 335 may have other configurations such as a square, rectangular, or other polygonal shapes that may enable the extended protrusion 335 to focally compress the EJV and/or IJV of the individual.

In various embodiments, when the actuation device 2 (165) is in the actuated state, the distance between the first end 312a and the second end 312b remains unchanged in comparison to the distance when the actuation device 2 (165) is in the rest state. In some embodiments, as is shown in FIG. 3C, the distance 375 between the first end 312a and the second end 312b decreases when the actuation device 2 (165) transitions to the actuated state.

In this embodiment, the housing 314 may be composed of a flexible material. As such, when transitioning from the rest state to the actuated state, the housing 314 of the actuation device 2 (165) can reduce its curvature such that the reduced distance 375 between the first end 312a and the second end 312b is decreased compared to the distance 370 shown in FIG. 3A. In some embodiments, the flexible material is an electroactive polymer such as a liquid crystalline polymer, or a piezoelectric polymer.

As shown in FIG. 3C, the actuation device 2 (165) need not include extendable protrusions 330 as was shown in FIG. 3A and 3B. In various embodiments, the first end 312a and the second end 312b each have vertical edges 350a and 350b, respectively. The vertical edges 350 of each of the first end 312a and second end 312b can physically contact the skin of the individual, thereby bilaterally compressing the IJV of the individual.

### EXAMPLE ACTUATION DEVICE FOR CLOSING THE PARAVERTEBRAL VENOUS PLEXUS

### EXAMPLE DEVICE FOR STIMULATING A GAG REFLEX

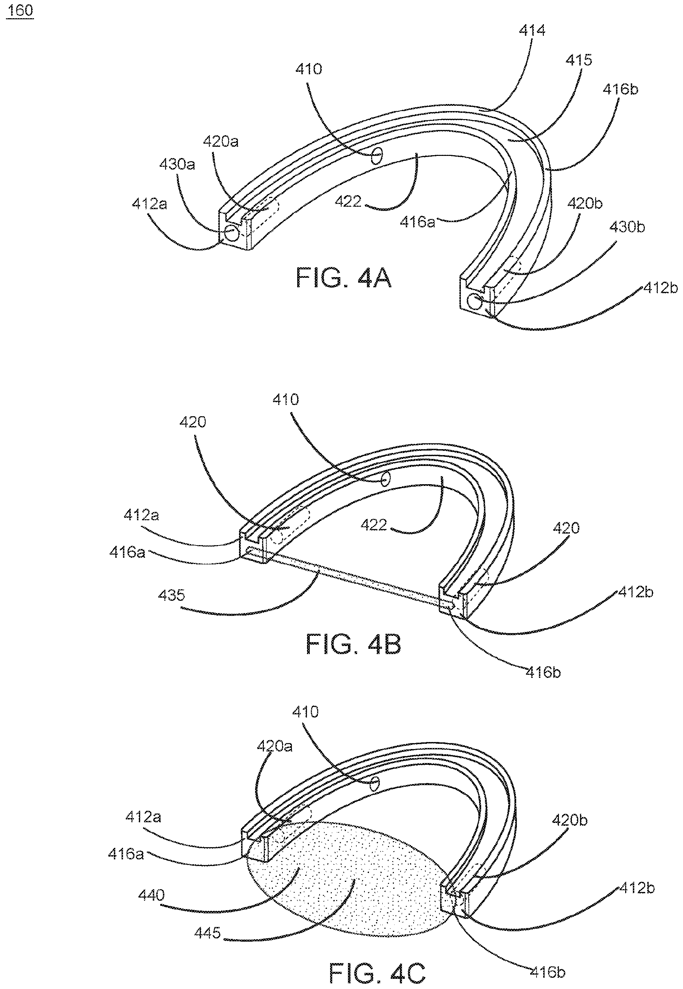

FIGs. 4A-C each illustrates an example actuation device 160 for inducing a gag reflex in an individual, in accordance with an embodiment. In one embodiment, a gag reflex can be invoked by the actuation device 1 (160) by physically contacting the actuation device 1 (160) with one or more various locations within the individual's mouth. For example, the stimulation structures of the actuation device 1 (160) may contact the back of the tongue, at or around the tonsils, the uvula, or the back of the throat. The physical contact can result in stimulation of the glossopharyngeal nerve, thereby resulting in a gag reflex. In other embodiments, a gag reflex can be invoked by the actuation device 1 (160) by directly electrically stimulating a nerve, such as the glossopharyngeal nerve, to cause the gag reflex.

Specifically, FIG. 4A depicts the actuation device 1 (160) in a rest state. The actuation device 1 (160) may be configured to be worn in a mouth of the individual. For example, as depicted in FIG. 4A, the actuation device 1 (160), is designed as a mouthguard device configured to reside within the mouth of the individual. Specifically, the actuation device 1 (160) may include a housing 414 that further includes a first end 412a and a second end 412b. The housing 414 may be contoured (e.g. curved) and may include features such as an inner ridge 416a, and outer ridge 416b, and a cavity 415 formed by the inner ridge 416a and outer ridge 416b. Additionally, the actuation device 1 (160) can include communication electronics 410, actuating components 420, and stimulation structures 430.

Referring first to the housing 414 of the actuation device 1 (160), as depicted in FIG. 4A, the housing 414 includes the inner ridge 416a and the outer ridge 416b. The inner ridge 416a forms the internal surface 422 of the actuation device 1 (160) and connects the first end 412a of the housing 414 to the second end 412b of the housing 414. The outer ridge 416b forms an external surface of the actuation device 1 (160) and similarly connects the first end 412a of the housing 414 to the second end 412b of the housing 414. A cavity 415 can be located between the inner ridge 416a and the outer ridge 416b. In other words, the cavity 415 is lined on either side by one of the inner ridge 416a or the outer ridge 416b. In some embodiments, the cavity 415 has a depth between 1 millimeter and 20 millimeters. Specifically, the depth of the cavity 415 can be designed such that the individual's teeth can comfortably reside in the cavity 415 when the actuation device 1 (160) is worn in the mouth. Furthermore, the cavity 415 (as well as the inner ridge 416a and outer ridge 416b) is shaped with a particular curvature. In various embodiments, the curvature may be determined based on the curvature of the individual's teeth.

As depicted in FIG. 4A, the cavity 415 is located on a top side of the actuation device 1 (160) and therefore, enables an individual's upper teeth to rest comfortably in the cavity 415 when the actuation device 1 (160) is worn. In various embodiments, the actuation device 1 (160) further includes a cavity (not shown) on the underside of the actuation device 1 (160). The cavity on the underside of the actuation device 1 (160) may be similarly configured as the cavity 415 on the top side of the actuation device 1 (160). As such, the cavity on the underside of the actuation device 1 (160) may similarly enable an individual's lower teeth to rest comfortably in the cavity when wearing the actuation device 1 (160). In some embodiments, the cavity 415 on either the top or underside of the actuation device 1 (160) includes additional features such as undulations or indentations that are designed to mimic the shape (e.g., gaps, indentations and the like) of the individual's upper or lower teeth.

Referring to the communication electronics 410 of the actuation device 1 (160), they enable the actuation device 1 (160) to communicate with the wearable device 155 through one of Bluetooth, near field communication (NFC), WiFi, 2G, 3G, 4G, LTE, or other wireless or wired communication methods. Specifically, the communication electronics 410 of the actuation device 1 (160) receives input from the wearable device 155 after a protective measure is deemed necessary. Therefore, the actuation device 1 (160) can transition from a rest state, as depicted in FIG. 4A, to an actuated state as is depicted in FIG. 4B and 4C. Alternatively, the communication electronics 410 can receive input from the wearable device 155 after an endpoint is detected. Therefore, the actuation device 1 (160) can transition from the actuated state back to the rest state. Although FIG. 4A depicts the communication electronics 410 as being located on the inner surface 422 of the actuation device 1 (160), in various embodiments, the communication electronics 410 can be located on another surface or internally within the housing 414 of the actuation device 1 (160).

The actuation device 1 (160) can include multiple actuating components 420a and 420b that are triggered when the communication electronics 410 of the actuation device 1 (160) receives the input indicating a need for a protective measure from the wearable device 155. As shown in FIG. 4A, a first actuating component 420a can be located in the housing 414 near or at the first end 412a whereas a second actuating component 420b can be located in the housing 414 near or at the second end 412b. In other embodiments, the actuating components 420 can be located within the housing 414 of the actuation device 1 (160) at other locations.

An actuating component 420 of the actuation device 1 (160) can provide one of a mechanical, chemical, or electrical inputs to actuate a stimulation structure 430. In other embodiments, input can be of a different energy modality such as an electromagnetic (e.g., magnetic) input. Specifically, in the embodiment shown in FIG. 4A, the actuating component 420 can provide an electrical input through the stimulation structure, also referred to as the electrodes 430. A first electrode 430a can be located at the first end 412a whereas a second electrode 430b can be located at the second end 412b. The electrodes 430 may be electrical contacts on a surface of the first end 412a and a surface of the second end 412b. Therefore, when actuated, an actuating component 420 can provide an input through the electrical contacts of the electrodes 430 in order to cause a gag reflex. Specifically, in reference to FIG. 4A, the actuating component 420 can be electrical hardware (e.g., battery, circuitry hardware) configured to provide an electrical input. The electrical contacts of the electrodes 430 can provide the electrical input (an applied voltage or an applied current) to the individual wearing the actuation device 1 (160).

In various embodiments, when residing in the mouth of the individual, the first end 412a and the second end 412b of the actuation device 1 (160) may each be in contact with a portion of the mouth of the individual. Specifically, the first end 412a and the second end 412b of the actuation device 1 (160) are in contact with one of the back of the tongue, the tonsils or areas around the tonsils, the uvula, or the back of the throat. Therefore, the electrode contacts of the electrodes 430 can similarly be in contact with one of the back of the tongue, the tonsils or areas around the tonsils, the uvula, or the back of the throat. The electrical input applied by the electrode 430 can trigger the glossopharyngeal nerve at the back of the individual's mouth to cause the gag reflex.

In various embodiments, the actuation device 1 (160) imparts a bilateral electrical stimulation through the two electrode contacts of the two electrodes 430. In some embodiments, a unilateral stimulation through a single electrode contact of a single electrode 430 is sufficient to cause a corresponding gag reflex in the individual.

Reference is now made to FIG. 4B, which illustrates a different embodiment of the actuation device 1 (160) for stimulating a gag reflex. Specifically, as shown in FIG. 4B, the actuation device 1 (160) is in a rest state. In this embodiment, the stimulation structure of the actuation device 1 (160) may be an inflatable structure 435 that causes a gag reflex in the individual. In various embodiments, this embodiment does not include electrode contacts of electrodes 430 as shown in FIG. 4A. In some embodiments, both the electrode contacts of the electrodes 430 and an inflatable structure 435 are included in the actuation device 1 (160) for stimulating a gag reflex.

The inflatable structure 435 may be coupled to the first end 412a of the actuation device 1 (160) through a first attachment point 416a. Furthermore, the inflatable structure 435 can be coupled to the second end 412b of the actuation device 1 (160) through a second attachment point 416b. Therefore, as shown in FIG. 4B, the inflatable structure 435 may directly traverse the distance between the first end 412a and the second end 412b of the actuation device 1 (160). In some embodiments, when in the rest state, the inflatable structure 435 can traverse along the inner surface 422 of the actuation device 1 (160). In other words, the inflatable structure 435 may follow the curvature of the inner surface 422 of the actuation device 1 (160) such that the inflatable structure 435 is less than a threshold distance away from the inner surface 422. Here, positioning the inflatable structure 435 close the inner surface 422 of the actuation device 1 (160) can prevent accidental stimulation of a gag reflex.

In various embodiments, the actuation device 1 (160) may include more than one inflatable structures 435. For example, a first inflatable structure 435 can be coupled at the first attachment point 416a to the first end 412a of the housing 414. Additionally, a second inflatable structure 435 can be coupled at the second attachment point 416b to the second end 412b of the housing 414. In some embodiments, when in the rest state, each of the first and second inflatable structure 435 may sit flush with the surface of the first end 412a and the surface of the second end 412b. For example, the first and second inflatable structure 435 can be stored in a cavity in the first end 412a and the second end 412b, respectively. Thus, this can prevent accidental stimulation of a gag reflex by the inflatable structures 435 when in a rest state.

FIG. 4C depicts the actuation device 1 (160) in an actuated state, in accordance with an embodiment. Here, the inflatable structure 435 shown in FIG. 4B changes its configuration to achieve an actuated inflated structure 440 (e.g., inflated). The actuated inflated structure 440 may remain coupled through the first attachment point 416a to the first end 412a of the housing 414 and through the second attachment point 416b to the second end 412b of the housing 414. When worn in the mouth of the individual, the actuated inflated structure 440 can physically contact one of the back of the tongue, the tonsils or areas around the tonsils, the uvula, or the back of the throat. As such, the actuated inflated structure 440 causes a gag reflex to occur in the individual.

In this embodiment, to achieve the actuated state, the actuating components 420 of the actuation device 1 (160) can cause the transition from the inflatable structure 435 shown in FIG. 4B to the actuated inflated structure 440 shown in FIG. 4C. As described above, the actuating components 420 can provide a chemical input. For example, each actuating component 420 can be configured to mix substances such as gasses, liquids, gels, and the like. Each substance can be stored within a cartridge that may be internal to the actuation device 1(160) or external to actuation device 1 (160). Mixing the substances causes a conversion of the substances into an expanded fluid, gas, or vapor that subsequently fills the inflatable structure 435. Altogether, the mixing of substances generates an expanded fluid, gas, or vapor that actuates (e.g., inflates) the inflatable structure 435 to achieve the actuated inflated structure 440 shown in FIG. 4C.

In some embodiments, the first actuating component 420a actuates a first inflatable structure 435 coupled to the first end 412a through attachment point 416a. Additionally, the second actuating component 420b actuates a second inflatable structure 435 coupled to the second end 412b through attachment point 416b. Therefore, the two inflatable structures may each actuate (e.g., inflate) and cause a gag reflex.

In various embodiments, the actuated inflated structure 440 (and the inflatable structure 435 when at rest) include vents 445 distributed throughout the actuated inflated structure 440 that enables a substance from within the actuated inflated structure 440 to escape. For example, the expanded fluid, gas, or vapor that actuated the inflatable structure 435 can escape through the vents 445 at a controlled rate depending on the size of the vents 445. As such, the actuated inflated structure 440 can return from the actuated state back to the inflatable structure 435 at a rest state. In another embodiment, the actuated inflated structure 440 includes a vent 445 that can be opened or closed given an electrical, chemical, or mechanical input. The input can be provided by the actuating component 420. As such, when the wearable device 155 detects that a protective measure is needed, the vent 445 can be closed when transitioning the actuation device 1 (160) from a rest state to an actuated state. Likewise, when the wearable device 155 detects an endpoint, vent 445 can be opened to transition the actuation device 1 (160) from an actuated state to a rest state.

### EXAMPLE DEVICE FOR STIMULATING A VALSALVA-LIKE MANEUVER

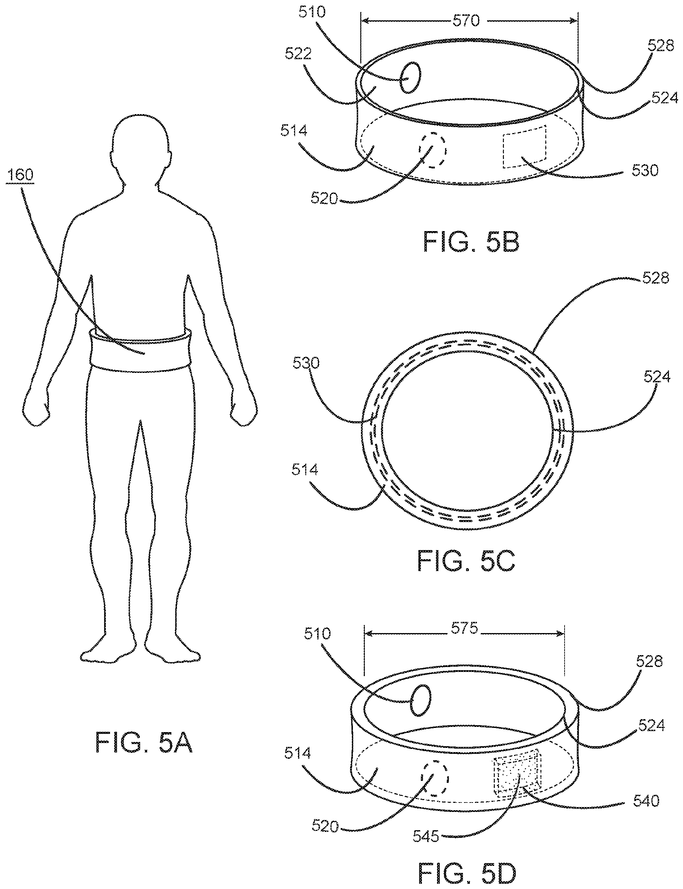

FIGs. 5A-C illustrates an example actuation device 1 (160) for inducing a valsalva-like maneuver, in accordance with an embodiment. For example, as depicted in FIG. 5A, the example actuation device 1 (160) is a waistband worn by an individual around his/her waist. In this embodiment, the waistband can be situated external to the individual's diaphragm. As such, when actuated, the waistband causes a valsalva-like maneuver by compressing the diaphragm and causing an increase in intraabdominal pressure and pressure within the thoracic cavity. In other embodiments, the example actuation device 1 (160) can be worn at any anatomical region between the chest and the waist. Although FIG. 5A depicts the waistband as fully encircling the individual's waist, in some embodiments, the waistband only encircles a portion of the individual. In some embodiments, the waistband encircles less than 50% of the individual's waist.

Referring to FIG. 5B, the actuation device 1 (160) may include communication electronics 510 and an actuating component 520 located within a housing 514 of the actuation device 1 (160). The actuation device 1 (160) may have an inner wall 524 and an outer wall 528. As such, the actuation device 1 (160) has inner diameter 570. Additionally, between the inner wall 524 and outer wall 528, the actuation device 1 (160) can include a stimulation structure such as an expandable cavity 530.

Similar to the communication electronics 410 described above in regards to FIG. 4, the communication electronics 510 of the actuation device 1 (160) can enable the actuation device 1 (160) to communicate with the wearable device 155 through one of Bluetooth, near field communication (NFC), WiFi, 2G, 3G, 4G, LTE, or other wireless or wired communication methods. Specifically, the communication electronics 510 of the actuation device 1 (160) receives input from the wearable device 155 after a protective measure is deemed necessary. Therefore, the actuation device 1 (160) can transition from a rest state, as depicted in FIG. 5B, to an actuated state as is depicted in FIG. 5D. Alternatively, the communication electronics 510 can receive input from the wearable device 155 after an endpoint is detected. Therefore, the actuation device 1 (160) can transition from the actuated state back to the rest state. In various embodiments, the communication electronics 510 can be located on the inner surface 522 of the actuation device 1 (160). In various embodiments, the communication electronics 510 can be located internally within the actuation device 1 (160).

The dimensions of the actuation device 1 (160) can be configured such that the actuation device 1 (160) be comfortably worn at the individual's waist when in the rest state. For example, the inner diameter 570 is designed so the inner wall 524 of the actuation device 1 (160) contacts, but does not constrict, the individual's waist. In various embodiments, the distance between the inner wall 524 and the outer wall 528 of the actuation device 1 (160) can range from 1 centimeter up to 10 centimeters when in the rest state.

The stimulation structure of the actuation device 1 (160), specifically an expandable cavity 530 as shown in FIG. 5B, is located between the inner wall 524 and the outer wall 528 of the actuation device 1 (160). In one embodiment, the expandable cavity 530 is only located within a portion of the actuation device 1 (160). As shown in FIG. 5B, the expandable cavity 530 may be a cavity that, when worn by the individual, is situated externally to the diaphragm. Therefore, actuation of the expandable cavity 530 would directly compress the diaphragm and cause a valsalva-like maneuver. FIG. 5C depicts a top-down view of another embodiment of the expandable cavity 530, as indicated by the dotted outlined structure, within the actuation device 1 (160) in the rest state. In this scenario, the expandable cavity 530 is concentrically located between the inner walls 524 and the outer walls 528 of the actuation device 1 (160).

Referring back to FIG. 5B, the actuating component 520 can cause the expandable cavity 530 to actuate (e.g., expand). As described above, the actuating component 520 can provide one or more of a mechanical, chemical, or electrical input to actuate the expandable cavity 530. As an example, each actuating component 530 can mix substances such as gasses, liquids, gels, and the like. The substances can be initially stored in a cartridge that is internal or external to the actuating device 1 (160). Mixing the substances causes a conversion of the substances into an expanded fluid, gas, or vapor that subsequently fills and expands the expandable cavity 530. The expansion of the expandable cavity 530 can happen in an instantaneous manner (e.g., less than 1 second). As such, the expandable cavity 530 can be actuated, as depicted in FIG. 5D.

FIG. 5D depicts the actuation device 1 (160) in an actuated state, in accordance with an embodiment. Generally, the actuated cavity 540 can cause the actuation device 1 (160) to alter its configuration and cause a valsalva-like maneuver. The distance between the inner wall 524 and the outer wall 528 can be increased in the actuated state as compared to the corresponding distance when in the rest state. Additionally, the inner diameter 575 of the actuation device 1 (160) can be reduced in the actuated state as compared to the inner diameter 570 of the actuation device 1 (160) when in the rest state. Although FIG. 5C depicts a reduced inner diameter 575 across the entire inner wall 524, in various embodiments, a reduced inner diameter 575 only occurs in a portion of the actuation device 1 (160). For example, if the expandable cavity 530 is within a portion of the actuation device 1 (160) as shown in FIG. 5B, then the reduced inner diameter 575 of the actuation device 1 (160) can correspond to the location of the inner wall 524 where the actuated cavity 540 is located.

In various embodiments, the actuated cavity 540 also include vents 545 distributed throughout the actuated cavity 540. The vents 545 may be connected through the inner wall 524 or outer wall 528 such that a substance from within the actuated cavity 540 can escape out to the environment. For example, the expanded fluid, gas, or vapor that previously actuated the expandable cavity 530 can escape through the vents 545 at a controlled rate depending on the size of the vents 545. As such, the actuated cavity 540 can return from the actuated state back to the expandable cavity 530 at a rest state. In another embodiment, the actuated cavity 540 includes a vent 545 that can be opened or closed given an electrical, chemical, or mechanical input. The input can be provided by the actuating component 520. As such, when the wearable device 155 detects that a protective measure is needed, the vent 545 can be closed when transitioning the actuation device 1 (160) from a rest state to an actuated state. Likewise, when the wearable device 155 detects an endpoint, vent 545 can be opened to transition the actuation device 1 (160) from an actuated state to a rest state.

### EXAMPLE COMPUTING SYSTEM OF THE SYSTEM ENVIRONMENT

FIG. 6 illustrates an example block diagram depicting the system architecture of a computing system 120, in accordance with an embodiment. The computing system 120 may include a data processing module 610, a model training module 620, and a model application module 630.

The data processing module 610 receives gathered sensor data. In one embodiment, the data processing module 610 receives gathered sensor data from the remote sensors 125 and/or from the sensors 180 of the wearable device 155. As previously described, the gathered sensor data can include orientation and/or position of the wearable device 155 as well as data corresponding to an occurring or impending collision (e.g., speed of collision). Additionally, if the system environment 100 includes multiple brain injury reduction systems 150, then the data processing module 610 can receive gathered sensor data corresponding to the relative location of a first wearable device from a first brain injury reduction system 150 to a second wearable device from a second brain injury reduction system 150. In this scenario, the computing system 120 can analyze collisions that may occur as a result of two or more wearable devices 155. This can be particularly relevant in analyzing collisions in a competitive sporting event such as a football game.

In one embodiment, the data processing module 610 may preprocess the gathered data such that the data can be subsequently analyzed. For example, the data processing module 610 can apply a filter (e.g., low pass, bandpass, or high pass) to eliminate noise in the gathered data. The processed data is then provided to either the model training module 620 for training a machine-learning model or to the model application module 630 for determination of whether a protective measure is needed.

The model training module 620 executes a machine learning algorithm using the received processed data as training data to train a machine learning model. Machine learning techniques for training the machine-learning model may be one of random forests, neural networks, naive Bayes, support vector machines, short-term memory networks, logistic regression, bagged trees, decision trees, boosted trees and machine learning used in HIVE^{™} frameworks, in different embodiments.

More specifically, the computing system 120 may train a classification model such that the classification model outputs one of two possible outputs. For example, given the training data, the classification model is trained by using, as output labels, a designation of whether a protective measure is needed or not. In some embodiments, a classification model is trained specifically for an individual. In other words, a classification model can be trained to consider the personal tendencies and behavior of an individual that may change the susceptibility of the individual to brain injury. In other embodiments, the model training module 620 trains a classification model for a group of individuals that is specific for a demographic (e.g., elderly individuals) or any other characteristic (e.g., for a particular football position such as a running back, a quarterback, a linesman, and the like).

The model application module 630 applies a trained machine-learning model to newly received sensor data that corresponds to a currently occurring or impending collision. Thus, the trained machine learning model evaluates whether a protective measure needs to be performed in response to the gathered data. If a protective measure is needed, the computing system 120 provides the evaluation to the brain injury reduction system 150. Therefore, the wearable device 155 of the brain injury reduction system 150 can execute the protective measure. In some embodiments, the computing system 120 provides the trained machine learning model to the brain injury reduction system 150 such that when an occurring or impending collision is detected, the wearable device 155 can rapidly determine whether a protective measure is to be provided by applying the trained machine learning model.

### PROVIDING A PROTECTIVE MEASURE FOR REDUCING BRAIN INJURY

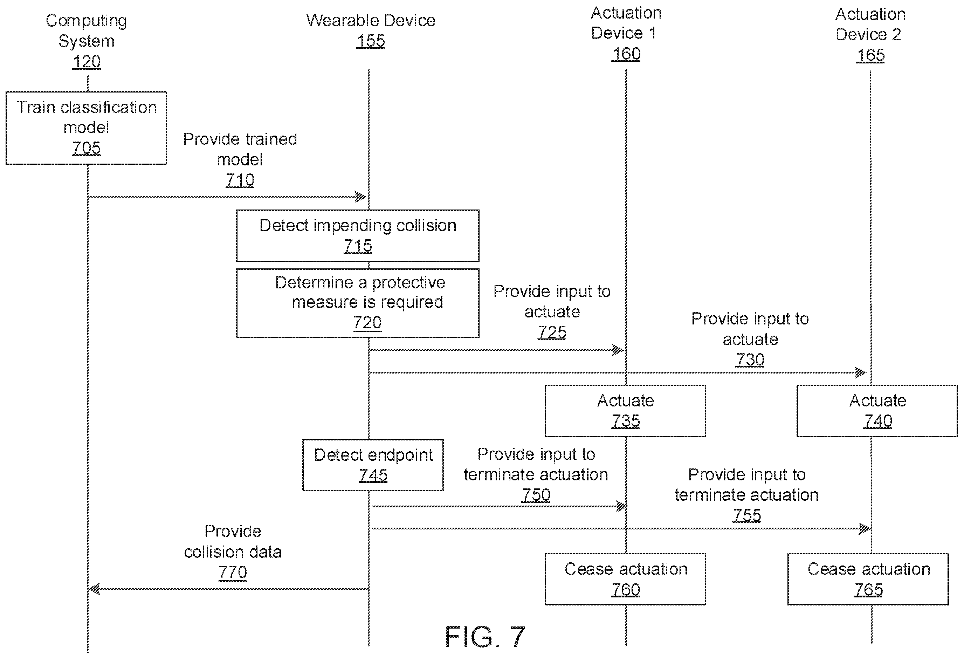

FIG. 7 illustrates an example interaction diagram for providing a protective measure for reducing brain injury in an individual, in accordance with an embodiment. In some embodiments, the process of providing a protective measure includes the wearable device 155 and one of the actuation devices 160 or 165. In various embodiments, the process need not include the computing system 120 such that detection of an impending collision by the wearable device 155 is performed autonomously.

Initially, a computing system 120 of the system environment 100 may train 705 a classification model that receives data corresponding to an occurring or impending collision as inputs, and outputs a classification as to whether a protective measure is to be provided. Generally, the classification model can be trained on training data that is derived from prior collisions. For example, training data may include data that is specific to an individual that was involved in the prior collision including, but not limited to, the orientation and/or position of the individual, the speed at which the individual was traveling, unique characteristics of the individual (e.g., reaction time), and the like. Therefore, a trained classification model may be specifically trained for a particular individual such that determination of whether a protective measure is required is specifically tailored for the individual.

As depicted in FIG. 7, the trained classification model is provided 710 to the wearable device 155 of the brain injury reduction system 150. Therefore, when the wearable device 155 detects 715 an impending collision, the wearable device 155 can rapidly determine 720 that a protective measure is required by applying the data corresponding to the impending collision to the trained classification model.

In another embodiment, the trained classification model is maintained by the computing system 120. Therefore, when the wearable device 155 detects 715 an impending collision, the data corresponding to the impending collision is transmitted to the computing system 120 to determine whether a protective measure is required. As such, the computing system 120 can provide an input to the wearable device 155 as to whether a protective measure is to be performed.

Once the wearable device 155 determines or receives instructions that a protective measure is required, the wearable device 155 provides 725 an input to an actuation device 1 (160) and provides 730 an input to an actuation device 2 (165). This input may be provided through wired or wireless communication technology (e.g., Bluetooth, NFC, WiFi, LTE, and the like). In some embodiments, the wearable device 155 only provides 730 an input to actuation device 1 (160) to maintain intracranial venous pressure by reducing venous drainage through the PVP.

Each of actuation device 1 (160) and actuation device 2 (165) actuate 735 and 740, thereby reducing venous drainage through the PVP and the IJVs, respectively. As previously described, actuation of actuation device 1 (160) may involve stimulating a gag reflex or a valsalva-like maneuver using actuation device 1 (160). Additionally or alternatively, actuation of actuation device 2 (165) may involve external compression of the IJVs through physical structures of the actuation device 2 (165).

The wearable device 155 detects 745 an endpoint. For example, a detected endpoint may be detection of collisions. The wearable device 155 can record data corresponding to the detected collisions. As another example, a detected endpoint may be a pre-determined duration of time after the protective measure is provided. In some embodiments, the endpoint is a detected one or more collisions followed by a pre-determined duration after the detected one or more collisions. The wearable device 155 provides 750 input to the actuation device 1 (160) to terminate actuation. Alternatively or additionally, the wearable device 155 provides 755 input to the actuation device 2 (165) to terminate actuation. As such, the actuation device 1 (160) and/or the actuation device 2 (165) return to a rest state in response to the input. In some embodiments, each of the actuation device 1 (160) and actuation device 2 (165) return to an original configuration. Therefore, the individual need not endure a prolonged period of the protective measure after the collision has occurred.

In various embodiments, the wearable device 155 can provide 770 recorded collision data (e.g., orientation of the individual, imparted force, speed of collision, and the like) to the computing system 120. As such, the computing system 120 can continue to train the classification model 705 to more accurately determine when a protective measure for the individual is required.

### ADDITIONAL EMBODIMENT CONSIDERATIONS

Throughout this specification, as used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus.

In addition, use of the "a" or "an" are employed to describe elements and components of the embodiments herein. This is done merely for convenience and to give a general sense of the invention. This description should be read to include one or at least one and the singular also includes the plural unless it is obvious that it is meant otherwise.

The term "housing" or "means for housing," as used throughout, may refer to any holder or structure that houses all or a portion of a component of the system, such as an actuation device, a sensor, or both. The term "actuation device" or "means for actuating" as used throughout, may refer to any device or entity (including mechanical, electrical, or chemical actuation devices) that interacts with the body or otherwise operates to cause a response that prevents or reduces outflow of venous drainage from the PVP, IJVs, or both (or other component via which venous drainage from the intracranial compartment occurs). The term "sensing device," "sensor," "means for detecting" or "means for sensing," as used throughout, may refer to any device capable of sensing or detecting an effect or occurrence that indicates an impending or occurring collision.

Finally, as used herein any reference to "one embodiment," "some embodiments," or "various embodiments" means that a particular element, feature, structure, or characteristic described in connection with the embodiment is included in at least one embodiment. The appearances of the phrase "in one embodiment" in various places in the specification are not necessarily all referring to the same embodiment.

Upon reading this disclosure, those skilled in the art will appreciate still additional alternative structural and functional designs as disclosed herein. Thus, while particular embodiments and applications have been illustrated and described, it is to be understood that the disclosed embodiments are not limited to the precise construction and components disclosed herein. Various modifications, changes and variations, which will be apparent to those skilled in the art, may be made in the arrangement and details of the apparatus disclosed herein without departing from the scope of the invention defined in the appended claims.

## Claims

1. A system for reducing a severity of a brain injury incurred by an individual due to a change in head acceleration of the individual, the system comprising:
one or more wearable housings (140, 205, 314, 414) configured to be worn on the body of the individual;
a wearable sensing device (155) comprising:
one or more sensors (180) on or within at least one of the housings (205), each of the one or more sensors (180) being positioned on the wearable sensing device (155) and configured to detect one of an occurring or impending collision that would result in the change in head acceleration;
a wearable actuation device (160, 165) comprising:
one or more stimulation structures (145, 330, 335) on or within at least one of the housings (140, 314), the stimulation structures (145, 330, 335) each configured to cause one of a gag reflex or a valsalva-like maneuver;
wherein the one or more stimulation structures (145) of the wearable actuation device (160) include electrodes (145) configured to be in contact with skin of an individual located above or near the thoraco-abdominal nerves, wherein the electrodes (145) are housed by electrode housings, wherein actuation of the wearable actuation device including the one or more stimulation structures (145) causes the valsalva-like maneuver as a result of stimulating the thoraco-abdominal nerves or the rectus abdominal muscles of the individual directly;
communication electronics (190a) on or within at least one of the housings (140, 205, 314) and configured to transmit an input from the wearable sensing device (155) to the wearable actuation device (160, 165), the input instructing the wearable actuation device (160,165) to initiate actuation to cause the valsalva-like maneuver subsequent to detection of the occurring or impending collision by the one or more sensors (180) of the wearable sensing device (155).

2. The system of claim 1, wherein a first set of communication electronics (190a) are located on the wearable sensing device (155) and wherein a second set of communication electronics (190a) are located on the wearable actuation device (160, 165).

3. The system of claim 1 or 2, wherein the wearable sensing device (155) is a helmet and wherein the one or more sensors (180) of the wearable sensing device (155) are on or within a housing (205) of the helmet.

4. The system of any one of claims 1-3, wherein one or more of the stimulation structures (145, 430) of the wearable actuation device (165) is an additional electrode (145, 430) configured to transcutaneously stimulate one of a phrenic nerve or a laryngeal nerve of the individual.

5. The system of any one of claims 1-2, wherein the wearable actuation device (160) includes a mouthguard configured to reside in the mouth of the individual.

6. The system of claim 5, wherein the wearable actuation device (160) further comprises:
a first end (412a) comprising a first additional electrode (430a);
a second end (412b) comprising a second additional electrode (430b); and
a contoured portion of at least one of the housings (414) comprising:
an external cavity (415) configured to receive teeth of the individual; and
an actuating component (420) for providing an electric input to the mouth of the individual through the first additional electrode (430a) of the first end (412a) and the second additional electrode (430b) of the second end (412b) of the actuation device (160).

7. The system of any one of claims 1-6, wherein the wearable actuation device (160) further comprises an inflatable balloon, wherein the inflatable balloon comprises vents (545) that are configured to vent a fluid from within the inflatable balloon.

8. The system of any one of claims 1-2, wherein the wearable actuation device (160) further comprises an expandable waistband having a first inner diameter when in a rest state and a second inner diameter when in an actuated state, wherein the second inner diameter is smaller than the first inner diameter.

9. The system of claim 8, wherein:
the expandable waistband is an inflatable cavity (530) located between an inner wall (524) and an outer wall (528) of the inflatable waistband; and/or
the expandable waistband comprises vents (545) in fluid connection with the inflatable cavity (530) that are configured to vent a fluid from within the inflatable cavity (530).

10. The system of any one of claims 1-9 further comprising:
a second wearable actuation device (165) comprising:
one or more stimulation structures (330) on or within at least one of the housings (314);
a first end (312a);
a second end (312b); and
a housing (314) configured to be circumferentially positioned around a neck of the individual, the housing (314) comprising a first extendable protrusion (330) and a second extendable protrusion (330),
wherein the first extendable protrusion (330) of the second wearable actuation device (165) is configured to compress a left internal jugular vein when in an actuated state, and
wherein the second extendable protrusion (330) is configured to compress a right internal jugular vein of the individual when in the actuated state.

11. The system of claim 10, wherein:
the first extendable protrusion (330) and the second extendable protrusion (330) of the second wearable actuation device (165)transforms into the actuated state in response to detection of the impending collision by the one or more sensors (180) of the wearable sensing device (155); and/or
the first extendable protrusion (330) of the second wearable actuation device (165) is located on an inner surface of the second wearable device (165), and wherein the second extendable protrusion (330) of the second wearable actuation device (165) is located on an inner surface of the second wearable device (165); and/or
the housing (314) of the second actuation device (165) has a larger curvature in an actuated state than when in a rest state.

12. The system of any one of claims 1-11 further comprising:
a computing system (120) in communication with the wearable sensing device (155) of the system, the computing system (120) configured to apply a trained machine learning model to data corresponding to the occurring or impending collision detected by the one or more sensors (180) of the wearable sensing device (155).

13. The system of any one of claims 1-12, wherein the system further comprises:
one or more remote sensors (125) in communication with the computing system and the wearable sensing device (155) of the system,
wherein the trained machine learning model was previously trained using training data obtained, at least in part, by the one or more remote sensors (125).

14. The system of any one of claims 1-13, wherein the communication electronics (190a) are configured to transmit an input from the wearable sensing device (155) to one or both of the first actuation device (160) and the second actuation device (165) to cease actuation.

15. The system of any one of claims 1-14, further comprising a processor (190b) configured to execute instructions that determine whether a protective measure is needed, comprising:
receive a trained machine-learning model from the computing system (120) to be used to determine whether a protective measure is needed;
analyze data from the wearable sensing device (155); output whether the protective measure is to be performed; and
in response to a determination by the machine learning model that the protective measure is needed, instruct the communication electronics (190a) to transmit the input to the wearable actuation device (160, 165).

16. The system of claim 1, wherein the electrodes (145) configured to be in contact with skin of an individual located above or near the thoraco-abdominal nerves is a first set of electrodes included in the one or more stimulation structures;
wherein the one or more stimulation structures further include a second set of electrodes (145) configured to be in contact with skin of an individual located external or near to the laryngeal nerves, wherein the second set of electrodes (145) are housed by electrode housings, wherein actuation of the wearable actuation device including the one or more stimulation structures (145) causes a gag reflex as a result of the second set of electrodes stimulating the laryngeal nerves;
wherein the input transmitted by the communication electronics (190a) instructs the wearable actuation device (160,165) to initiate actuation to cause the gag reflex and the valsalva-like maneuver subsequent to detection of the occurring or impending collision by the one or more sensors (180) of the wearable sensing device (155).

## Patentansprüche

1. System zum Verringern eines Schweregrads einer Hirnverletzung, die bei einem Individuum aufgrund einer Kopfbeschleunigungsänderung hervorgerufen wurde, wobei das System Folgendes umfasst:
ein oder mehrere tragbare Gehäuse (140, 205, 314, 414), die dazu ausgelegt sind, an dem Körper des Individuums getragen zu werden;
eine tragbare Sensorvorrichtung (155), die Folgendes umfasst:
einen oder mehrere Sensoren (180) auf oder in zumindest einem aus den Gehäusen (205), wobei jeder aus dem einen oder den mehreren Sensoren (180) auf der tragbaren Sensorvorrichtung (155) positioniert und dazu ausgelegt ist, eines aus einem stattfindenden oder einem bevorstehenden Aufprall zu detektieren, der zu der Kopfbeschleunigungsänderung führen würde;
eine tragbare Aktuatorvorrichtung (160, 165), umfassend:
eine oder mehrere Stimulierstrukturen (145, 330, 335) auf oder in zumindest einem aus den Gehäusen (140, 314), wobei die Stimulierstrukturen (145, 330, 335) jeweils dazu ausgelegt sind, eines aus einem Würgereflex oder einem Valsalva-ähnlichen Manöver auszulösen;
wobei die eine oder mehreren Stimulierstrukturen (145) der tragbaren Aktuatorvorrichtung (160) Elektroden (145) umfassen, die dazu ausgelegt sind, mit der Haut eines Individuums oberhalb oder nahe der thorakoabdominalen Nerven in Berührung zu sein, wobei die Elektroden (145) durch Elektrodengehäuse eingehaust sind, wobei das Aktivieren der tragbaren Aktuatorvorrichtung, welche die eine oder mehreren Stimulierstrukturen (145) umfasst, das Valsalva-ähnliche Manöver als eine Folge des direkten Stimulierens der thorakoabdominalen Nerven oder der Nerven des geraden Bauchmuskels des Individuums auslöst;
eine Kommunikationselektronik (190a) auf oder in zumindest einem aus den Gehäusen (140, 205, 314), die dazu ausgelegt ist, eine Eingabe von der tragbaren Sensorvorrichtung (155) an die tragbare Aktuatorvorrichtung (160, 165) zu übertragen, wobei die Eingabe die tragbare Aktuatorvorrichtung (160, 165) anweist, eine Aktivierung zu initiieren, um das Valsalva-ähnliche Manöver nach dem Detektieren des stattfindenden oder bevorstehenden Aufpralls durch den einen oder die mehreren Sensoren (180) der tragbaren Sensorvorrichtung (155) auszulösen.

2. System nach Anspruch 1, wobei ein erster Satz der Kommunikationselektronik (190a) auf der tragbaren Sensorvorrichtung (155) angeordnet ist und wobei ein zweiter Satz der Kommunikationselektronik (190a) auf der tragbaren Aktuatorvorrichtung (160, 165) angeordnet ist.

3. System nach Anspruch 1 oder 2, wobei die tragbare Sensorvorrichtung (155) ein Helm ist und wobei der eine oder die mehreren Sensoren (180) der tragbaren Sensorvorrichtung (155) auf oder in einem Gehäuse (205) des Helms sind.

4. System nach einem der Ansprüche 1 bis 3, wobei eine oder mehrere aus den Stimulierstrukturen (145, 430) der tragbaren Aktuatorvorrichtung (165) eine zusätzliche Elektrode (145, 430) sind, welche dazu ausgelegt ist, eines aus einem Zwerchfellnerv oder einem Kehlkopfnerv des Individuums transkutan zu stimulieren.

5. System nach einem der Ansprüche 1 bis 2, wobei die tragbare Aktuatorvorrichtung (160) einen Mundschutz umfasst, der dazu ausgelegt ist, im Mund des Individuums zu sitzen.

6. System nach Anspruch 5, wobei die tragbare Aktuatorvorrichtung (160) ferner Folgendes umfasst:
ein erstes Ende (412a), umfassend eine erste zusätzliche Elektrode (430a);
ein zweites Ende (412b), umfassend eine zweite zusätzliche Elektrode (430b); und
einen konturierten Abschnitt von zumindest einem aus den Gehäusen (414), umfassend:
einen äußeren Hohlraum (415), der dazu ausgelegt ist, Zähne des Individuums aufzunehmen; und
eine Aktuatorkomponente (420) zum Bereitstellen eines elektrischen Eingangs in den Mund des Individuums über die erste zusätzliche Elektrode (430a) des ersten Endes (412a) und die zweite zusätzliche Elektrode (430b) des zweiten Endes (412b) der Aktuatorvorrichtung (160).

7. System nach einem der Ansprüche 1 bis 6, wobei die tragbare Aktuatorvorrichtung (160) ferner einen aufblasbaren Ballon umfasst, wobei der aufblasbare Ballon Lüftungsöffnungen (545) umfasst, die dazu ausgelegt sind, ein Fluid aus dem aufblasbaren Ballon auszulassen.

8. System nach einem der Ansprüche 1 bis 2, wobei die tragbare Aktuatorvorrichtung (160) ferner einen erweiterbaren Taillenbund umfasst, der in einem Ruhezustand einen ersten Innendurchmesser aufweist und in einem aktivierten Zustand einen zweiten Innendurchmesser aufweist, wobei der zweite Innendurchmesser kleiner als der erste Innendurchmesser ist.

9. System nach Anspruch 8, wobei:
der erweiterbare Taillenbund ein aufblasbarer Hohlraum (530) ist, der zwischen einer Innenwand (524) und einer Außenwand (528) des aufblasbaren Taillenbunds angeordnet ist; und/oder
der erweiterbare Taillenbund Lüftungsöffnungen (545) in Fluidverbindung mit dem aufblasbaren Hohlraum (530) aufweist, die dazu ausgelegt sind, ein Fluid aus dem Inneren des aufblasbaren Hohlraums (530) auszulassen.

10. System nach einem der Ansprüche 1 bis 9, ferner umfassend:
eine zweite tragbare Aktuatorvorrichtung (165), umfassend:
eine oder mehrere Stimulierstrukturen (330) auf oder in zumindest einem aus den Gehäusen (314);
ein erstes Ende (312a);
ein zweites Ende (312b); und
ein Gehäuse (314), das dazu ausgelegt ist, umfangsmäßig um den Hals des Individuums angeordnet zu sein, wobei das Gehäuse (314) einen ersten erweiterbaren Vorsprung (330) und einen zweiten erweiterbaren Vorsprung (330) umfasst,
wobei der erste erweiterbare Vorsprung (330) der zweiten tragbaren Aktuatorvorrichtung (165) dazu ausgelegt ist, in einem aktivierten Zustand eine linke innere Halsvene zusammenzudrücken, und
wobei der zweite erweiterbare Vorsprung (330) dazu ausgelegt ist, im aktivierten Zustand eine rechte innere Halsvene des Individuums zusammenzudrücken.

11. System nach Anspruch 10, wobei:
der erste erweiterbare Vorsprung (330) und der zweite erweiterbare Vorsprung (330) der zweiten tragbaren Aktuatorvorrichtung (165) sich als Reaktion auf das Detektieren des bevorstehenden Aufpralls durch den einen oder die mehreren Sensoren (180) der tragbaren Sensorvorrichtung (155) in den aktivierten Zustand verwandelt; und/oder
der erste erweiterbare Vorsprung (330) der zweiten tragbaren Aktuatorvorrichtung (165) auf einer Innenseite der zweiten tragbaren Vorrichtung (165) angeordnet ist und wobei der zweite erweiterbare Vorsprung (330) der zweiten tragbaren Aktuatorvorrichtung (165) auf einer Innenseite der zweiten tragbaren Vorrichtung (165) angeordnet ist; und/oder
das Gehäuse (314) der zweiten Aktuatorvorrichtung (165) in einem aktivierten Zustand eine stärkere Krümmung aufweist als in einem Ruhezustand.

12. System nach einem der Ansprüche 1 bis 11, ferner umfassend:
ein Computersystem (120) in Kommunikation mit der tragbaren Sensorvorrichtung (155) des Systems, wobei das Computersystem (120) konfiguriert ist, um ein trainiertes maschinelles Lernmodell auf Daten anzuwenden, die dem stattfindenden oder bevorstehenden Aufprall, welcher durch den einen oder die mehreren Sensoren (180) der tragbaren Sensorvorrichtung (155) detektiert wurde, entsprechen.

13. System nach einem der Ansprüche 1 bis 12, wobei das System ferner Folgendes umfasst:
einen oder mehrere entfernte Sensoren (125) in Kommunikation mit dem Computersystem und der tragbaren Sensorvorrichtung (155) des Systems,
wobei das trainierte maschinelle Lernmodell zuvor unter Verwendung von Trainingsdaten trainiert wurde, die zumindest teilweise unter Verwendung des einen oder der mehreren entfernten Sensoren (125) erhalten wurden.

14. System nach einem der Ansprüche 1 bis 13, wobei die Kommunikationselektronik (190a) dazu ausgelegt ist, eine Eingabe von der tragbaren Sensorvorrichtung (155) an eine oder beide aus der ersten Aktuatorvorrichtung (160) und der zweiten Aktuatorvorrichtung (165) zu übertragen, um die Aktivierung zu beenden.

15. System nach einem der Ansprüche 1 bis 14, ferner umfassend einen Prozessor (190b), der konfiguriert ist, um Befehle auszuführen, die bestimmen, ob eine Schutzmaßnahme erforderlich ist, umfassend:
Empfangen eines trainierten maschinellen Lernmodells vom Computersystem (120), um verwendet zu werden, um zu bestimmen, ob eine Schutzmaßnahme erforderlich ist;
Analysieren von Daten von der tragbaren Sensorvorrichtung (155);
Ausgeben, ob die Schutzmaßnahme durchzuführen ist; und
als Reaktion auf das Bestimmen, dass die Schutzmaßnahme erforderlich ist, durch das maschinelle Lernmodell, Anweisen der Kommunikationselektronik (190a), die Eingabe an die tragbare Aktuatorvorrichtung (160, 165) zu übertragen.

16. System nach Anspruch 1, wobei die Elektroden (145), die dazu ausgelegt sind, mit der Haut eines Individuums oberhalb oder nahe der thorakoabdominalen Nerven in Berührung zu sein, ein erster Satz von Elektroden sind, der in der einen oder den mehreren Stimulierstrukturen umfasst ist;
wobei die eine oder mehreren Stimulierstrukturen ferner einen zweiten Satz von Elektroden (145) umfassen, die dazu ausgelegt sind, mit der Haut eines Individuums außerhalb oder nahe der Kehlkopfnerven in Berührung zu sein, wobei der zweite Satz von Elektroden (145) durch Elektrodengehäuse eingehaust ist, wobei das Aktivieren der tragbaren Aktuatorvorrichtung, welche die eine oder mehreren Stimulierstrukturen (145) umfasst, einen Würgereflex als eine Folge des Stimulierens der Kehlkopfnerven durch den zweiten Satz von Elektroden auslöst;
wobei die durch die Kommunikationselektronik (190a) übertragene Eingabe die tragbare Aktuatorvorrichtung (160, 165) anweist, die Aktivierung zu initiieren, um den Würgereflex und das Valsalva-ähnliche Manöver nach dem Detektieren des stattfindenden oder bevorstehenden Aufpralls durch den einen oder die mehreren Sensoren (180) der tragbaren Sensorvorrichtung (155) auszulösen.

## Revendications

1. Système permettant de réduire la gravité d'une lésion cérébrale subie par un individu en raison d'un changement d'accélération de la tête de l'individu, le système comprenant :
un ou plusieurs boîtiers pouvant être portés (140, 205, 314, 414) configurés pour être portés sur le corps de l'individu ;
un dispositif de détection pouvant être porté (155) comprenant :
un ou plusieurs capteurs (180) sur ou dans au moins un des boîtiers (205), chacun des un ou plusieurs capteurs (180) étant positionné sur le dispositif de détection pouvant être porté (155) et configuré pour détecter une collision en cours ou imminente susceptible d'entraîner le changement d'accélération de la tête ;
un dispositif d'actionnement pouvant être porté (160, 165) comprenant :
une ou plusieurs structures de stimulation (145, 330, 335) sur ou dans au moins un des boîtiers (140, 314), les structures de stimulation (145, 330, 335) étant chacune configurées pour provoquer un réflexe pharyngé ou une manœuvre de type manœuvre de Valsalva ;
dans lequel les une ou plusieurs structures de stimulation (145) du dispositif d'actionnement pouvant être porté (160) comportent des électrodes (145) configurées pour être en contact avec la peau d'un individu située au-dessus ou à proximité des nerfs thoraco-abdominaux, dans lequel les électrodes (145) sont logées dans des boîtiers d'électrodes, dans lequel l'actionnement du dispositif d'actionnement pouvant être porté comportant les une ou plusieurs structures de stimulation (145) provoque la manœuvre de type manœuvre de Valsalva à la suite d'une stimulation directe des nerfs thoraco-abdominaux ou du muscle grand droit de l'abdomen de l'individu ;
des composants électroniques de communication (190a) sur ou dans au moins un des boîtiers (140, 205, 314) et configurés pour transmettre une entrée en provenance du dispositif de détection pouvant être porté (155) au dispositif d'actionnement pouvant être porté (160, 165), l'entrée ordonnant au dispositif d'actionnement pouvant être porté (160, 165) d'initier un actionnement pour provoquer la manœuvre de type manœuvre de Valsalva suite à la détection de la collision en cours ou imminente par les un ou plusieurs capteurs (180) du dispositif de détection pouvant être porté (155).

2. Système selon la revendication 1, dans lequel un premier ensemble de composants électroniques de communication (190a) est situé sur le dispositif de détection pouvant être porté (155) et dans lequel un second ensemble de composants électroniques de communication (190a) est situé sur le dispositif d'actionnement pouvant être porté (160, 165).

3. Système selon la revendication 1 ou 2, dans lequel le dispositif de détection pouvant être porté (155) est un casque et dans lequel les un ou plusieurs capteurs (180) du dispositif de détection pouvant être porté (155) sont sur ou dans un boîtier (205) du casque.

4. Système selon l'une quelconque des revendications 1 à 3, dans lequel une ou plusieurs des structures de stimulation (145, 430) du dispositif d'actionnement pouvant être porté (165) sont une électrode supplémentaire (145, 430) configurée pour stimuler de manière transcutanée un nerf phrénique ou un nerf laryngé de l'individu.

5. Système selon l'une quelconque des revendications 1 à 2, dans lequel le dispositif d'actionnement pouvant être porté (160) comporte un protège-dents configuré pour résider dans la bouche de l'individu.

6. Système selon la revendication 5, dans lequel le dispositif d'actionnement pouvant être porté (160) comprend en outre :
une première extrémité (412a) comprenant une première électrode supplémentaire (430a) ;
une seconde extrémité (412b) comprenant une seconde électrode supplémentaire (430b) ; et
une partie profilée d'au moins un des boîtiers (414) comprenant :
une cavité externe (415) configurée pour recevoir les dents de l'individu ; et
un composant d'actionnement (420) permettant de fournir une entrée électrique à la bouche de l'individu par le biais de la première électrode supplémentaire (430a) de la première extrémité (412a) et de la seconde électrode supplémentaire (430b) de la seconde extrémité (412b) du dispositif d'actionnement (160).

7. Système selon l'une quelconque des revendications 1 à 6, dans lequel le dispositif d'actionnement pouvant être porté (160) comprend en outre un ballonnet gonflable, dans lequel le ballonnet gonflable comprend des évents (545) qui sont configurés pour évacuer un fluide de l'intérieur du ballonnet gonflable.

8. Système selon l'une quelconque des revendications 1 à 2, dans lequel le dispositif d'actionnement pouvant être porté (160) comprend en outre une ceinture extensible présentant un premier diamètre interne lorsqu'elle est dans un état de repos et un second diamètre interne lorsqu'elle est dans un état actionné, dans lequel le second diamètre interne est plus petit que le premier diamètre interne.

9. Système selon la revendication 8, dans lequel :
la ceinture extensible est une cavité gonflable (530) située entre une paroi interne (524) et une paroi externe (528) de la ceinture gonflable ; et/ou
la ceinture extensible comprend des évents (545) en communication fluidique avec la cavité gonflable (530) qui sont configurés pour évacuer un fluide de l'intérieur de la cavité gonflable (530).

10. Procédé selon l'une quelconque des revendications 1 à 9 comprenant en outre :
un second dispositif d'actionnement pouvant être porté (165) comprenant :
une ou plusieurs structures de stimulation (330) sur ou dans au moins un des boîtiers (314) ;
une première extrémité (312a) ;
une seconde extrémité (312b) ; et
un boîtier (314) configuré pour être positionné de manière circonférentielle autour du cou de l'individu, le boîtier (314) comprenant une première saillie extensible (330) et une seconde saillie extensible (330),
dans lequel la première saillie extensible (330) du second dispositif d'actionnement pouvant être porté (165) est configurée pour comprimer une veine jugulaire interne gauche lorsqu'elle est dans un état actionné, et
dans lequel la seconde saillie extensible (330) est configurée pour comprimer une veine jugulaire interne droite de l'individu lorsqu'elle est dans l'état actionné.

11. Système selon la revendication 10, dans lequel :
la première saillie extensible (330) et la seconde saillie extensible (330) du second dispositif d'actionnement pouvant être porté (165) se transforment en l'état actionné en réponse à la détection de la collision imminente par les un ou plusieurs capteurs (180) du dispositif de détection pouvant être porté (155) ; et/ou
la première saillie extensible (330) du second dispositif d'actionnement pouvant être porté (165) est située sur une surface interne du second dispositif pouvant être porté (165), et dans lequel la seconde saillie extensible (330) du second dispositif d'actionnement pouvant être porté (165) est située sur une surface interne du second dispositif pouvant être porté (165) ; et/ou
le boîtier (314) du second dispositif d'actionnement (165) présente une courbure plus grande dans un état actionné que dans un état de repos.

12. Procédé selon l'une quelconque des revendications 1 à 11 comprenant en outre :
un système informatique (120) en communication avec le dispositif de détection pouvant être porté (155) du système, le système informatique (120) étant configuré pour appliquer un modèle d'apprentissage automatique entraîné à des données correspondant à la collision en cours ou imminente détectée par les un ou plusieurs capteurs (180) du dispositif de détection pouvant être porté (155).

13. Système selon l'une quelconque des revendications 1 à 12, dans lequel le système comprend en outre :
un ou plusieurs capteurs à distance (125) en communication avec le système informatique et le dispositif de détection pouvant être porté (155) du système,
dans lequel le modèle d'apprentissage automatique entraîné a été précédemment entraîné à l'aide de données d'entraînement obtenues, au moins en partie, par les un ou plusieurs capteurs à distance (125).

14. Système selon l'une quelconque des revendications 1 à 13, dans lequel les composants électroniques de communication (190a) sont configurés pour transmettre une entrée provenant du dispositif de détection pouvant être porté (155) à l'un ou aux deux du premier dispositif d'actionnement (160) et du second dispositif d'actionnement (165) pour mettre un terme à l'actionnement.

15. Système selon l'une quelconque des revendications 1 à 14, comprenant en outre un processeur (190b) configuré pour exécuter des instructions qui déterminent si oui ou non une mesure de protection est nécessaire, consistant à :
recevoir un modèle d'apprentissage automatique entraîné en provenance du système informatique (120) à utiliser pour déterminer si oui ou non une mesure de protection est nécessaire ;
analyser les données provenant du dispositif de détection pouvant être porté (155) ; déterminer si oui ou non la mesure de protection doit être réalisée ; et
en réponse à une détermination par le modèle d'apprentissage automatique que la mesure de protection est nécessaire, ordonner aux composants électroniques de communication (190a) de transmettre l'entrée au dispositif d'actionnement pouvant être porté (160, 165).

16. Système selon la revendication 1, dans lequel les électrodes (145) configurées pour être en contact avec la peau d'un individu située au-dessus ou à proximité des nerfs thoraco-abdominaux constituent un premier ensemble d'électrodes inclus dans les une ou plusieurs structures de stimulation ;
dans lequel les une ou plusieurs structures de stimulation comportent en outre un second ensemble d'électrodes (145) configuré pour être en contact avec la peau d'un individu située à l'extérieur ou à proximité des nerfs laryngés, dans lequel le second ensemble d'électrodes (145) est logé par des boîtiers d'électrodes, dans lequel l'actionnement du dispositif d'actionnement pouvant être porté comportant les une ou plusieurs structures de stimulation (145) provoque un réflexe pharyngé à la suite de la stimulation des nerfs laryngés par le second ensemble d'électrodes ;
dans lequel l'entrée transmise par les composants électroniques de communication (190a) ordonne au dispositif d'actionnement pouvant être porté (160, 165) d'initier un actionnement pour provoquer le réflexe pharyngé et la manœuvre de type manœuvre de Valsalva suite à la détection de la collision en cours ou imminente par les un ou plusieurs capteurs (180) du dispositif de détection pouvant être porté (155).
